# EUROPEAN PATENT APPLICATION

(11) **EP 1 462 105 A1**
(43) Date of publication of application: **29.09.2004**
(21) Application number: 03024405.7
(22) Date of filing: 22.10.2003
(51) Int. Cl.: A61K 31/519, A61K 31/345, A61P 9/10

(54) **Activation specific inhibitors of NF-kB and method of treating inflammatory processes in cardio-vascular diseases**

(30) Priority: 28.03.2003 EP 03007159
(71) Applicant: Procorde GmbH, 82152 Martinsried (DE)
(72) Inventor: Münch, Götz, 80799 München (DE); Holthoff, Hans-Peter, 82402 Seeshaupt (DE); Ungerer, Martin, 82166 Gräfelfing (DE)
(74) Representative: Hartz, Nikolai F., Dr.

(57) **Abstract**

A 5H-Thiazolo[3,2]pyrimidine derivative or a salt thereof for use as a medicine, which is represented by the following formula (A): 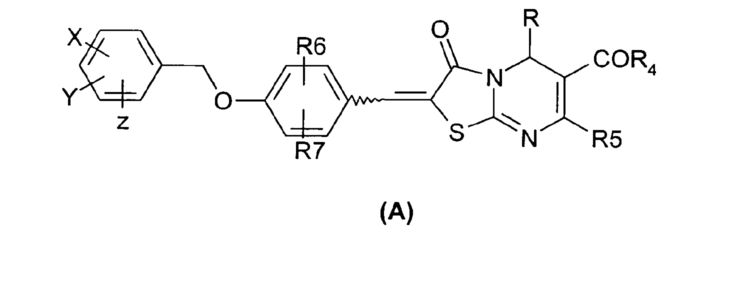 wherein
- R: represents an optionally substituted phenyl or pyridyl group;
- R₄: represents
a hydroxyl group,
an amino group,
a straight chain or branched alkoxy group,
a straight chain or branched alkyl group,
a cycloalkyloxy group,
an alkylamino group,
a cycloalkylamino group,
a dialkylamino group,
- R₅: represents
a hydrogen atom,
a straight chain or branched alkyl group,
- R₆ and R₇: which may be the same or different represent
a hydrogen atom,
a halogen atom,
a straight chain or branched alkyl group,
a straight chain or branched alkoxy group,
a straight chain or branched alkenyl group,
a cycloalkyl group,
an aryl group,
- X, Y, and Z: which may be same or different represent
a hydrogen atom,
a halogen atom,
a carboxyl group
a nitro group,
a cyano group,
an alkyl group,
an alkoxy group or
an acyl group.

## Description

### FIELD OF THE INVENTION

The present invention relates to the compounds for the activation specific inhibition of NF-kB, pharmaceutical compositions containing the compounds as active agents and the use of the compounds for the preparation of a medicine for the treatment or prevention of cardiovascular disease, in particular atherosclerosis.

### BACKGROUND OF THE INVENTION

Certain thiazolo[2,3-a]pyrimidine-6-carboxylic acids are known from DATABASE CHEMCATS [online] Chemical Abstracts Service, Columbus, Ohio, US; XP002247262 and INTERCHIM, Montlucon, Cedex, France, Publication Date: 09.07.2002; Catalog Name: INTERCHIM INTERMEDIATES. A medical application of these compounds is not known from this document.

Tozkoparan B., *et al.* disclose in *Arch. Pharm. Pharm. Med. Chem.* 331, 201-206, (1998) the synthesis and antiinflammatory activity of certain thiazolo[2,3-*a*]pyrimidines. The document, however, does not mention any possible mechanism of action of these antiinflammatory compounds. Ertan M., *et al*. disclose in *Arch. Pharm. (Weinheim)* 324, 135-139 (1991 ) the synthesis of 2-thioxo-1,2,3,4-tetrahydropyrimidine derivatives useful as intermediate compounds in the synthesis of thiazolo[2,3-a]pyrimidines.

WO 01/30774 and Hehner, S. P. *et al. Journal of Immunology* 163(10), 5617-5623 relate to inhibitors of NF-kB activation by compounds structurally unrelated to the compounds of the present invention.

Different toxic processes play a role for the initiation of atherosclerosis, of which cholesterol and other lipids are the most important factors. However, these processes occur in the age of early adolescence and have only limited pathological relevance for the complications of atherosclerosis. More important for the prognosis and the manifestation of the disease is the progress of the atherosclerotic alterations in middle aged and older patients. Atheroprogression is mainly driven by inflammatory processes in the endothelium, which are in turn maintained by different risk factors of atherosclerosis such as smoking, hypertension, hyperlipidemia and diabetes. The chronic inflammation in atherosclerosis is perpetuated by specific cytokines recruiting leukocytes to lesions, thus inducing the vicous circle of the inflammatory state within the arterial vessel wall [Ross, R, *N. Engl. J. Med.* 340:115-126, 1999].

The mechanism by which this chronic inflammatory process occurs is initially triggered by platelet endothelial interactions [Ross, R, *N. Engl. J. Med.* 340:115-126, 1999]. When platelets are activated they are known to release cytokines and growth factors into their surrounding environment [Gawaz M, *Circulation.* 96:1809-1818; 1997]. Adhesion of platelets to the endothelial surface is observed early in the atherogenic process and is associated with the release of biologically active molecules, e.g. IL-1 beta [Gawaz M, *Atherosclerosis.* 148:75-85; 2000] or CD40 ligand [Henn V; *Nature.* 391:591-594; 1998]. Early atherosclerosis is further characterized by adhesion of monocytes to endothelium and accumulation in the intimal layer, accompanied by foam cell formation. These activated platelets are able to induce activation of the transcription factor NF-kB in cultured endothelial cells [Gawaz, M; *Circulation.* 98:1164-1171; 1998]. Besides platelets, other prominent endothelial surface receptors with crucial roles for the perpetuation of atherosclerosis have the NF-kB system as common signalling pathway e.g. LOX-1 Receptors or toll-like receptors [Metha JL & L;D,J. Am.Coll.Cardiol. 39:1429-1435; 2002; Dunne; A & O'Neill LA; Science STKE 2003 (171):re 37].

NF-kB is an ubiquitous transcription factor of particular importance in mediating early inflammatory response genes such as MCP-1 [Bäuerle, P; *Cell.* 87:13-20; 1996], a C-C chemokine which is a potent chemoattractant for monocytes and abundant in atherosclerotic tissue [Neiken ; *J. Clin. Invest.* 88:1121-1127; 1991]. In unstimulated cells, NF-kB is found in the cytoplasm as a dimer, most frequently p50/p65, bound to inhibitory IkB proteins, e. g. IB-alpha, -beta and -epsilon, which prevent it from entering the nucleus. When cells are stimulated by cytokines, microbial products or oxidative stress, specific kinases phosphorylate IkB, causing its rapid ubiquitin-dependent proteolytic degradation by proteasomes. The release of NF-kB from IkB results in the passage of NF-kB into the nucleus, where it binds to specific kB sequences in promoter or enhancer regions thereby activating transcription of target genes involved in inflammatory, immunological, growth and apoptotic processes [Bäuerle, P; *Cell.* 87:13-20; 1996].

A key role for the signaling events that lead to the phosphorylation of IkB, plays the IkB kinase (IKK) complex, that has recently been identified [Karin ; *Annu. Rev. Immunol.* 18:621-663; 1996]. Prototypically, this complex contains two kinase-active components, namely IKK-alpha and IKK-beta as well as a kinase-inactive adaptor protein called IKK-gamma, which may be involved in stabilization of the complex or aid in its regulation. The function of IKK-alpha remains unclear, but it has been suggested to play a role in differentiation and proliferation whereas IKK-beta is regarded as the major IkB phosphorylating kinase, and is involved in proinflammatory and apoptotic processes [Karin ; *Annu. Rev. Immunol.* 18:621-663; 1996].

Activated platelets induce a transient activation of the endothelial I complex leading to proteolysis of IkB-alpha and -epsilon, similar to the effects seen after IL-1 beta stimulation. IKK-beta was identified as the most important kinase from the IKK complex and overexpression of a dominant-negative mutant form of IKK-beta substantially reduced platelet-induced IkB- and MCP-1 promoter-dependent transcription as well as MCP-1 secretion in endothelial cells [Gawaz, M; Thromb Haemost. 2002 Aug;88(2):307-14; 2002]. This resulted in a marked decrease of adhesion proteins VCAM and ICAM in endothelial cells. The surface expression of these adhesion proteins is increased on the endothelium of atherosclerotic animal models and humans. Moreover, these adhesion proteins play an important role for the increased sticking of inflammatory cells to the endothelium and furtherone invasion of monocytes into the endothelium. These monocytes further differentiate to macrophages and further perpetuate the inflammation in atheroprogression. Thus inhibition of this signalling by inhibition of IKK-beta disrupts a key step in the inflammation pathway in atheroprogression.

Different approaches to inhibit NF-kB activity have been described. One example is this inhibition of the association of IKK-gamma/NEMO signalsome complex [May, M.J; *Science* 289:1550-1554; 2000]. The assembly of the signalsome complex is essential for efficient phosphorylation of IkB and consecutive inhibtion of NF-kB. Another approach is the inhibition of the katalytic domain of the kinases for NF-kB inhibiton. As the NF-kB system is ubiquitous and responsible for various cellular processes, it would de desirable to specifically inhibit an overshooting NF-kB activity in inflammatory processes without affecting the basal activity.

### SUMMARY OF THE INVENTION

It is the problem of the invention to provide compounds with the specific capability to inhibit the activated form of NF-kB without affecting the basal activity for the treatment of inflammatory processes in atheroprogression. The use of such compounds for the production of medicaments, especially for the control or prevention of acute and/or chronic cardiovascular disorders of the aforementioned kind, is also an object of the invention.

This problem is solved according to the claims. The present invention solves an important problem for the treatment of atherosclerosis. The compounds of the invention inhibit the NF-kB pathway thereby treating or preventing the chronic inflammatory process in atherosclerotic arteries. The class of compounds inhibits specifically the activated form of the NF-kB system, which is predominantly found in atherosclerosis [Brand K, et al; J Clin Invest. 1996;97(7):1715-22] without affecting the basal NF-kB activity. The novel principle is not targeting the active domain of the NF-kB regulating kinases, but targets the stability of the signalsome complex of IKK-alpha and IKK-beta with NEMO. The integrity of this complex is essential for sufficient IkB phosphorylation and consecutive activation of NF-kB [May M. et al.; Science 289: 1550-1553; 2000]. Moreover, the compounds have specific proteolytic activity for the signalsome complex with the highest proteolytic activity for NEMO and weaker for IKK-alpha and IKK-beta. Other proteins independent from the NF-kB signalsome complex are not affected by this proteolytic activity. Direct inhibition of the kinase IKK-beta or IKK-alpha has severe detrimental effects such as induction of apoptisis with severe liver dysfunction [Li et al; Science 284 : 321-325; 1999] or immunosupressive side effects [Lavon I et al; Nature Medicine 6 (5); 573-577; 2000]. Therefore the concept of treatment of the inflammatory process in atherosclerosis by inhibition of the katayltic domain of NF-kB relating kinases is flawed by detrimental side effects which would not allow systemic application in patients.

Therefore we provide the solution of the problem of potent inhibition of the NF-kB mediated inflammation in atheroprogression without the deleterious side effect of complete NF-kB inhibition.

A first general aspect of the present invention relates to a 5H-Thiazolo[3,2]pyrimidine derivative or a salt or ester thereof for use as a medicine, which is represented by the following formula (A): 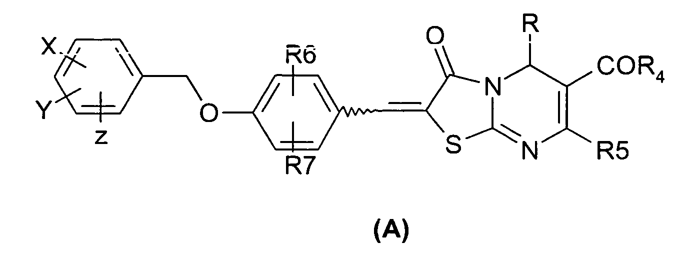 wherein
R represents an optionally substituted phenyl or pyridyl group;
R₄ represents
   a hydroxyl group,
   an amino group,
   a straight chain or branched alkoxy group,
   a straight chain or branched alkyl group,
   a cycloalkyloxy group,
   an alkylamino group,
   a cycloalkylamino group,
   a dialkylamino group,
R₅ represents
   a hydrogen atom,
   a straight chain or branched alkyl group,
R₆ and R₇ which may be the same or different represent
   a hydrogen atom,
   a halogen atom,
   a straight chain or branched alkyl group,
   a straight chain or branched alkoxy group,
   a straight chain or branched alkenyl group,
   a cycloalkyl group,
   an aryl group,
X, Y, and Z which may be same or different represent
   a hydrogen atom,
   a halogen atom,
   a carboxyl group
   a nitro group,
   a cyano group,
   an alkyl group,
   an alkoxy group or
   an acyl group.

A second general aspect of the present invention relates to a 5H-Thiazolo[3,2]pyrimidine derivative or a salt or ester thereof for use as a medicine, which is represented by the following formula (B): 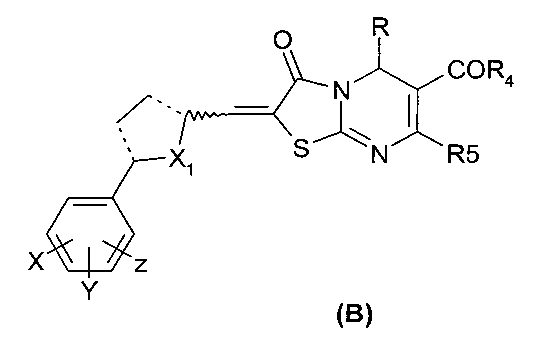 wherein
the dotted lines independently represent a single bond or a double bond;
R represents an optionally substituted phenyl or pyridyl group;
R₄ represents
   a hydroxyl group,
   an amino group,
   a straight chain or branched alkoxy group,
   a straight chain or branched alkyl group,
   a cycloalkyloxy group,
   an alkylamino group,
   a cycloalkylamino group,
   a dialkylamino group,
R₅ represents
   a hydrogen atom,
   a straight chain or branched alkyl group,
X₁ represents O, S, or NH;
X, Y, and Z which may be same or different represent
   a hydrogen atom,
   a halogen atom,
   a carboxyl group
   a nitro group,
   a cyano group,
   an alkyl group,
   an alkoxy group or
   an acyl group.

In formulae (A) and (B), the phenyl or pyridyl group represented by R may be substituted by 1 to 3 substituents selected from the group consisting of a halogens such as fluorine, chlorine, bromine and iodine, a cyano group, a hydroxy group, a nitro group, a carboxyl group, an amino group, a straight chain or branched C₁₋₆ alkyl group, a straight chain or branched C₁₋₆ alkoxy group, a straight chain or branched C₁₋₇ alkylcarbonyl group, a straight chain or branched C₁₋₇ alkoxycarbonyl group, straight chain or branched C₁₋₇ alkoxycarbonyloxy group, a straight chain or branched C₁₋₆alkylamino group, a straight chain or branched di-C₁₋₆alkylamino group, a straight chain or branched C₁₋₇ alkylcarbonylamino group, a straight chain or branched C₁₋ ₆alkylaminocarbonyl group, a straight chain or branched C₁₋₇ alkoxycarbonylamino group, a straight chain or branched C₁₋₇ alkylaminocarbonyloxy group. The phenyl or pyridyl group may also be substituted by an alkylenedioxy group such as a dioxmethylene, dioxyethylene, or dioxypropylene group. In formulae (A) and (B), the exocyclic double bond may be in the E or Z configuration. The present invention relates to both isomeric forms as well as mixtures therof. In formula (B), the dotted lines are preferably both double bonds.

A third general aspect of the present invention relates to a compound represented by the following formula (C): 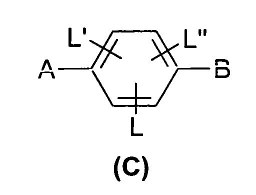 wherein
A and B which may be the same or different are a hydroxy group, a nitro group, a carboxyl group, an amino group, a straight chain or branched C₁₋₆ alkyl group, a straight chain or branched C₁₋₆ alkoxy group, a straight chain or branched C₁₋₇ alkylcarbonyl group, straight chain or branched C₁₋₇ alkoxycarbonyl group or a straight chain or branched C₁₋₆ alkylamino group, or represented by the following formula (1-1) 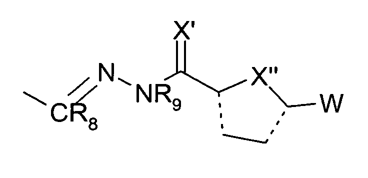
wherein
the dotted lines independently represent a single bond or a double bond,
R₈ and R₉ independently represent a hydrogen atom or a C₁₋₆ alkyl group,
X'and X" are independently O or S,
W is a hydrogen atom, nitro group, a cyano group, a carboxyl group or a group of the formula -COZ'R₁₀,
   wherein
   Z' is O or S or NH, and
   R₁₀ is a C₁₋₆ alkyl group; and
L, L' and L" which may be the same or different represent
   a hydrogen atom,
   a hydroxy group,
   an alkyl group,
   an alkoxy group,
   a halogen atom,
   a carboxyl group,
   an alkylcarbonyl group,
   an alkoxycarbonyl group,
   an amino group,
   an alkylamino group, or
   a dialkylamino group,
provided that at least one of A and B is represented by formula (1-1).

In a preferred class of compounds, A is hydrogen or a hydroxyl group. In a preferred class L, L', and L" are the same ore different and represent a hydrogen atom, a hydroxy group,an alkyl group,an alkoxy group, or a halogen atom. In a further preferred class of compounds A is hydrogen and at least one of L, L', and L" is a hydroxyl group. X' and X" are preferably oxygen atoms. Moreover, W is preferably a nitro group or a hydrogen atom. In a preferred class of compounds the dotted lines both represent a double bond.

A first aspect of the present invention relates to a 5H-Thiazolo[3,2]pyrimidine derivative or a salt or ester thereof for use as a medicine, which is represented by the following formula (I): 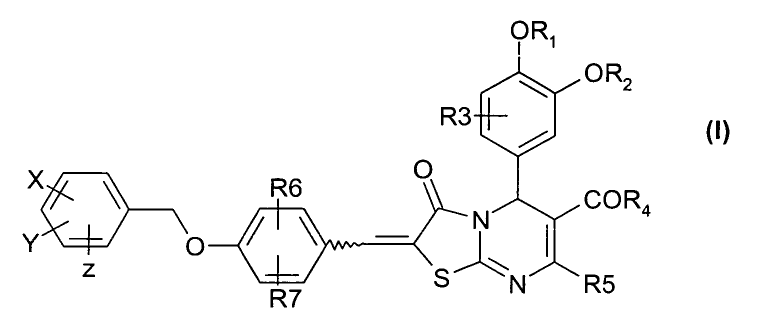 wherein
R₁ and R₂ which may be the same or different, represent
   a straight chain or branched alkyl group,
   a straight chain or branched alkenyl group,
   a cycloalkyl group,
   an aryl group, or
   R₁ and R₂ together may form an alkylene group;
R₃ represents
   a hydrogen atom,
   a halogen atom,
   a straight chain or branched alkyl group,
R₄ represents
   a straight chain or branched alkoxy group,
   a straight chain or branched alkyl group,
   a cycloalkyloxy group,
   an alkylamino group,
   a cycloalkylamino group,
R₅ represents
   a hydrogen atom,
   a straight chain or branched alkyl group,
R₆ and R₇ which may be the same or different represent
   a hydrogen atom,
   a halogen atom,
   a straight chain or branched alkyl group,
   a straight chain or branched alkoxy group,
   a straight chain or branched alkenyl group,
   a cycloalkyl group,
   an aryl group,
X, Y, and Z which may be same or different represent
   a hydrogen atom,
   a halogen atom,
   an alkyl group,or
   an acyl group.

A second aspect of the present invention relates to a 5H-Thiazolo[3,2]pyrimidine derivative or a salt or ester thereof for use as a medicine, which is represented by the following formula (II): 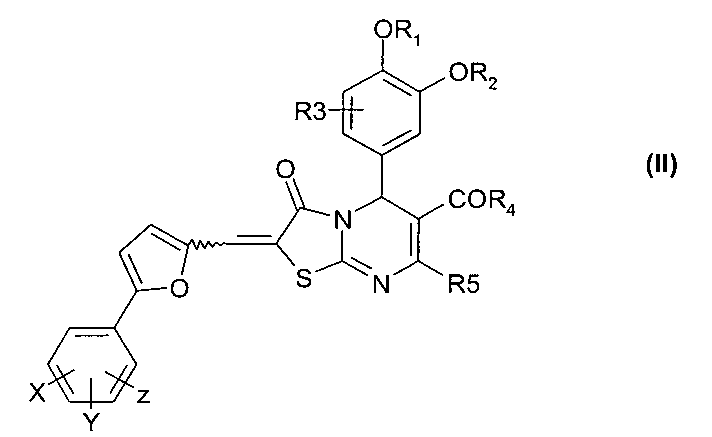 wherein
R₁ and R₂ which may be the same or different, represent
   a straight chain or branched alkyl group,
   a straight chain or branched alkenyl group,
   a cycloalkyl group,
   an aryl group, or
   R₁ and R₂ together may form an alkylene group;
R₃ represents
   a hydrogen atom,
   a halogen atom,
   a straight chain or branched alkyl group,
R₄ represents
   a straight chain or branched alkoxy group,
   a straight chain or branched alkyl group,
   a cycloalkyloxy group,
   an alkylamino group,
   a cycloalkylamino group,
R₅ represents
   a hydrogen atom,
   a straight chain or branched alkyl group,
X, Y, and Z which may be same or different represent
   a hydrogen atom,
   a carboxyl group
   a halogen atom,
   a nitro group,
   a cyano group, or
   an acyl group.

A third aspect of the present invention relates to a compound for use as a medicine, or a salt or ester thereof, wherein the compound is represented by the following formula (III): 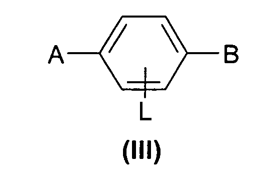 wherein
A and B which may be the same or different are represented by the following formula 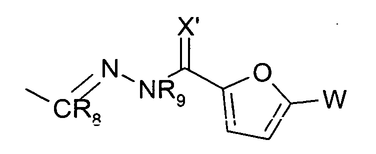 wherein
R₈ and R₉ independently represent a hydrogen atom or a C₁₋₆ alkyl group,
X' is O or S,
W is a nitro group, a cyano group, a carboxyl group or a group of the formula - COZ'R₁₀,
   wherein
   Z' is O or S or NH, and
   R₁₀ is a C₁₋₆ alkyl group; and
L represents
   a hydrogen atom,
   an alkyl group,
   an alkoxy group, or
   a halogen atom.

Compounds according to a further aspect of the invention give a positive result as an inhibitor in a cell-free screening method for the identification of inhibitors of IkB phosphorylation by IKK-β, whereby the method comprises the following steps:
(a) providing a composition containing a functional IKK-complex;
(b) subjecting a substrate peptide comprising IKK-β phosphorylation domain of IkB of in the presence of the compound to phosphorylation by the functional IKK-complex of step (a) under predetermined conditions;
(c) reacting the phosphorylated substrate peptide of step (b) under predetermined conditions with an antibody specific for the IKK-β phosphorylated domain of the stubstrate peptide,
(d) identifying the compound as an inhibitor when the amount of specifically bonded antibody is lower due to the presence of the compound as compared to the absence of the compound.

Compounds according to a further aspect of the invention give a positive result as an inhibitor in a cell assay for the identification of inhibitors of IkB phosphorylation by IKK-β, which assay comprises the following steps:
(a) providing a cell culture;
(b) subjecting the cells in the cell culture to TNFα in the presence of a test compound;
(c) isolating functional IKK-complex by immunoprecipitation using an anti-IKK-NEMO antibody;
(d) subjecting a substrate peptide comprising IKK-β phosphorylation domain of IkB to phosphorylation by the functional IKK-complex of step (c) under predetermined conditions;
(e) reacting the phosphorylated substrate peptide of step (d) with an antibody specific for the phosphorylated domain of the stubstrate peptide,
(f) identifying a test compound as an inhibitor when the amount of specifically bonded antibody is lower due to the presence of the test compound as compared to the absence of the test compound.

The present invention also provides a pharmaceutical composition comprising the compound as an active ingredient for reducing the activity of NF-κB.

### DESCRIPTION OF THE PREFERRED EMBODIMENTS

COM and corn in conjunction with a number designates a compound shown with its chemical structure in this specification.

The compounds according to the present invention are represented by the formulae (A), (B), (C), (I), (II), and (III). In the formulae an alkyl group can include linear or branched alkyl groups having 1 to 6 carbon atoms, preferably 1 to 4 carbon atoms, for example, methyl, ethyl, n-propyl, isopropyl, n-butyl, isobutyl, sec-butyl, tert-butyl, n-pentyl, isopentyl and n-hexyl. Examples of the alkenyl group can include linear or branched alkenyl groups having 2 to 6 carbon atoms, preferably 2 to 4 carbon atoms and 1 to 2 double bonds, for example, ethenyl, propenyl, butenyl, isobutenyl and butadienyl. Examples of the cycloalkyl group can include those having 3 to 6 carbon atoms, for example, cyclopropyl, cyclobutyl, cyclopentyl and cyclohexyl. Examples for an alkoxy group can include linear or branched alkoxy groups having 1 to 6 carbon atoms, preferably 1 to 4 carbon atoms, for example, methoxy, ethoxy, n-propoxy, isopropoxy, n-butoxy, isobutoxy, sec-butoxy, tert-butoxy, n-pentoxy, isopentoxy and n-hexoxy. Examples of the cycloalkyloxy group can include those having 3 to 6 carbon atoms, for example, cyclopropyloxy, cyclobutyloxy, cyclopentyloxy and cyclohexyloxy. Examples of the halogen atoms include fluorine chlorine, bromine and iodine. In the formulae, illustrative of the aryl group can be phenyl, naphthyl and pyridyl, with phenyl and pyridyl being particularly preferred. Examples of the alkylene group can be a linear or branched one having 1 to 6 carbon atoms, with one having 1 to 4 carbon atoms being preferred. Illustrative can be methylene, ethylene, and trimethylene. Examples for an alkylamino group can include an amino group having one or two substituents selected from linear or branched alkyl groups having 1 to 6 carbon atoms, preferably 1 to 4 carbon atoms, for example, methyl, ethyl, n-propyl, isopropyl, n-butyl, isobutyl, sec-butyl, tert-butyl, n-pentyl, isopentyl and n-hexyl. Examples for a dialkylamino group can include an amino group having two substituents selected from linear or branched alkyl groups having 1 to 6 carbon atoms, preferably 1 to 4 carbon atoms, for example, methyl, ethyl, n-propyl, isopropyl, n-butyl, isobutyl, sec-butyl, tert-butyl, n-pentyl, isopentyl and n-hexyl. Examples of the cycloalkylamino group can include those having 3 to 6 carbon atoms, for example, cyclopropylamino, cyclobutylamino, cyclopentylamino and cyclohexylamino. Examples of the acyl group can include acyl groups having 2 to 7 carbon atoms, preferably 2 to 5 carbon atoms. The carboxyl group may be in the form of a pharmaceutically acceptable metal salt, such as an alkali metal salt or an aline earth metal salt.

The above groups, in particular the aryl group, may contain 1 to 3 substituents. Examples of such substituents can include halogen atoms, C₁₋₄ alkyl groups, C₁₋₄ alkoxy groups, C₁₋₄ alkylthio groups, C₁₋₄ alkylsulfinyl groups, C₁₋₄ alkylsulfonyl groups, carboxyl group, C₂₋₅ alkoxycarbonyl groups, nitro group, amino group, and C₁₋₄ alkylamino groups. Here, illustrative of the halogen atoms can be fluorine, chlorine, bromine and iodine. The C₁₋₄ alkyl groups are, for example, methyl, ethyl, n-propyl, isopropyl and n butyl. Illustrative of the C₁₋₄ alkoxy groups are, for example, methoxy, ethoxy and propoxy. Illustrative of the C₁₋₄ alkylthio groups are, for example, methylthio, ethylthio and propylthio. Illustrative of the C₁₋₄ alkylsulfinyl groups are for example, methylsulfinyl, ethylsulfinyl and propylsulfinyl. Illustrative of the C₁₋₄ alkylsulfonyl groups are, for example, methylsulfonyl, ethylsulfonyl and propylsulfonyl. Illustrative of the C₂₋₅ alkoxycarbonyl groups can be those having alkoxy groups each of which contains 1 to 4 carbon atoms, for example, methoxycarbonyl, ethoxy carbonyl and propoxycarbonyl. Illustrative of the C₁₋₈ alkylamino groups can be those having one or two alkyl groups each of which contains 1 to 4 carbon atoms, for example, methylamino, dimethylamino, ethyl amino and propylamino. The alkyl moieties in these substituents may be linear, branched or cyclic.

The compounds of the invention may also contain common protecting groups for functional groups such as carboxyl groups. Moreover, the compounds of the invention may also be in the form of a prodrug which may be converted to an active agent under physiological conditions.

Preferred as R¹ and R² is an alkyl group, in particular a methyl or ethyl group, or an alkylene group, preferably a methylene or ethylene group. Preferred as R³ is a hydrogen atom or a halogen atom in particular in position 2 of the aromatic ring. Preferred as R⁴ is an alkoxy group, in particular a methoxy, ethoxy or propoxy group. Preferred as R⁵ is an alkyl group, in particular a mehyl, ethyl or propyl group. Preferred as R⁶ and R⁷ are an alkyl group, in particular a methoxy or ethoxy group, or a halogen atom, in particular iodine. Preferred as X, Y, or Z is a hydrogen atom or a halogen atom.

A preferred class of compounds of the general formula (I) are compounds wherein R¹ and R² together form an alkylene group, in particular a methylene or ethylene group; R³ is a hydrogen atom, R⁴ is an alkoxy group, in particular a methoxy, ethoxy or propoxy group, R⁵ is an alkyl group, in particular a methyl, ethyl or propyl group, R⁶ and R⁷ are an alkyl group, in particular a methoxy or ethoxy group, or a halogen atom, in particular iodine, X and Y are hydrogen atoms and Z is a halogen atom.

The most preferred compound of the general formula (I) may be represented by the following formula:

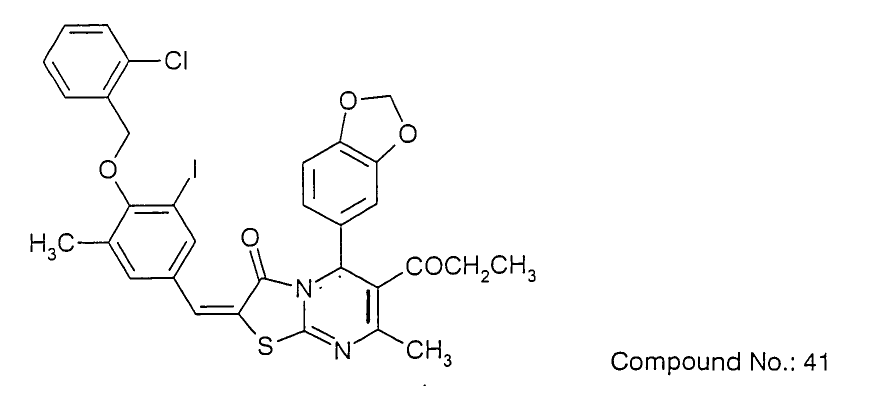

A preferred class of compounds of general formula (B) are those wherein R is a substituted phenyl group. The substituent is preferably a halogen atom, in particular a chlorine atom. A further preferred class of compounds of formula (B) or (II) consists of compounds wherein X is a substituent in meta-position, preferably a carboxyl group.

A preferred class of compounds of the general formula (II) are those wherein R¹ and R² is an alkyl group, in particular a methyl or ethyl group, R³ is a halogen atom in particular in position 2 of the aromatic ring, R⁴ is an alkoxy group, in particular a methoxy, ethoxy or propoxy group, R⁵ is an alkyl group, in particular a methyl, ethyl or propyl group, X, Y are hydrogen atoms and Z is a carboxyl group, preferably in position 3 or 4 of the aromatic ring.

The most preferred compounds of general formula (B) and (II) may be represented by the following formula

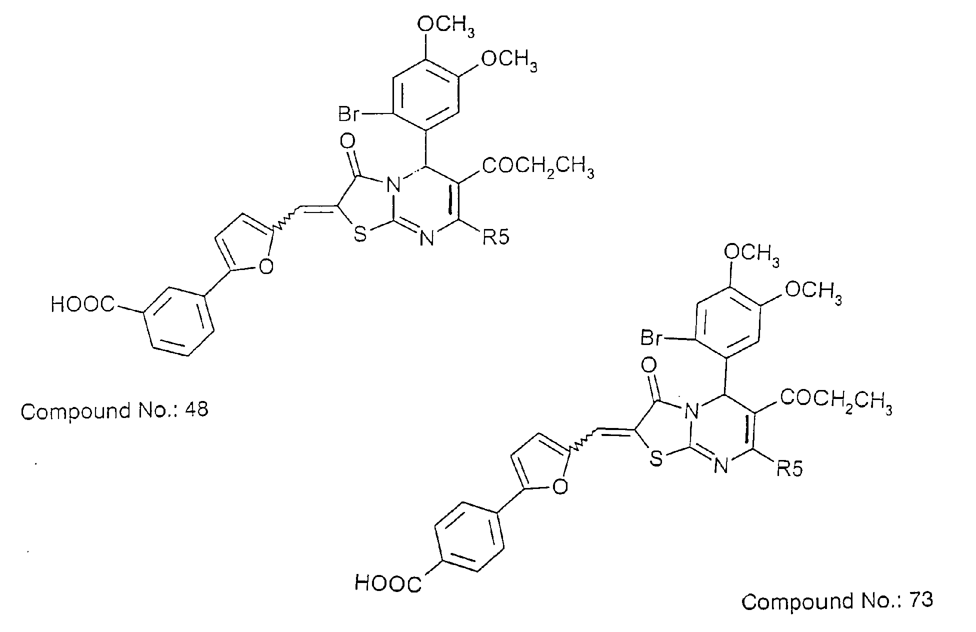

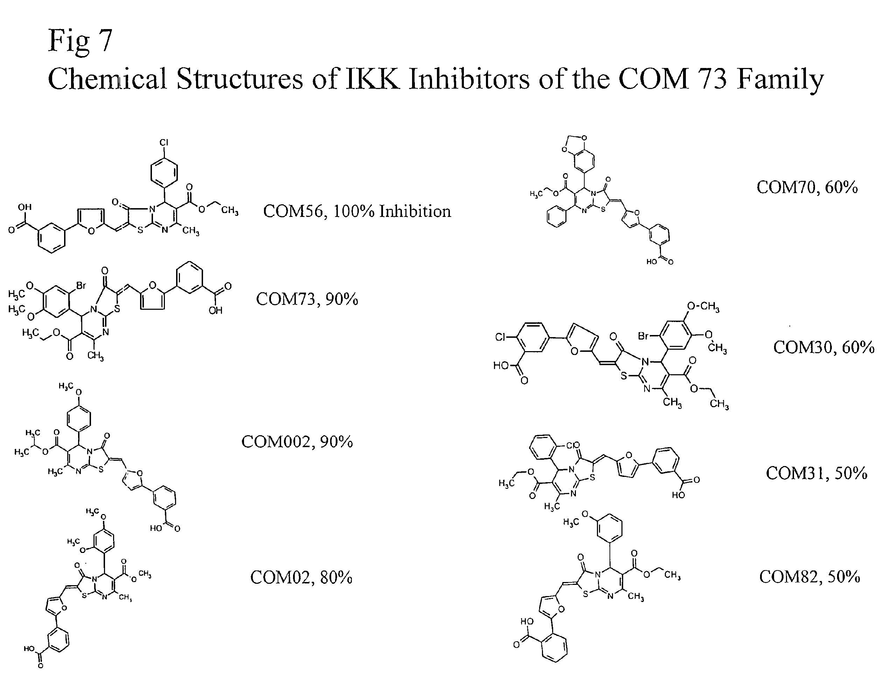

Further preferred embodiments of compounds of general formula (B) are shown in Figure 7.

A preferred class of compounds of the general formula (C) wherein A is hydrogen. In this group X' and X" are preferably oxygen atoms, L is a hydroxyl group, and W is a nitro group. In a further group of compounds, B is an alkyl or alkoxy group, preferably an alkoxy group, and L and W are hydrogen.

A preferred class of compounds of the general formula (III) are those wherein A and B are the same groups, R⁸ and R⁹ are the same or different and represent an alkyl group, in particular a methyl or ethyl group or a hydrogen atom, X' is an oxygen atom, L is a hydrogen atom and W is a nitro group.

The most preferred compounds of formula (C) and (III) may be represented by the following formula:

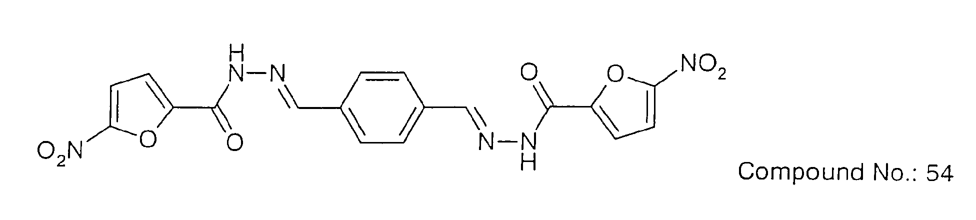

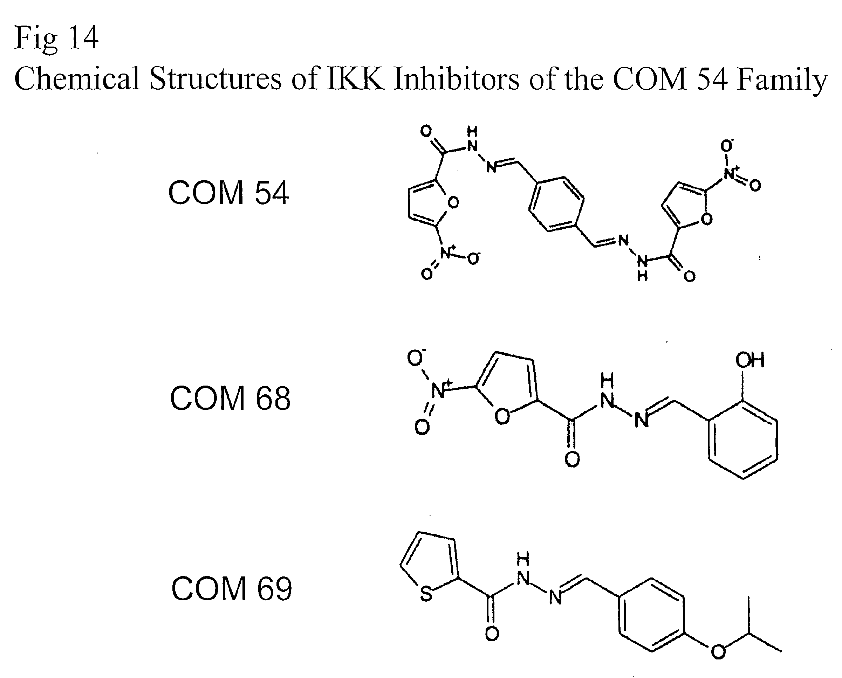

Further preferred compounds of formula (C) are shown in Fig. 14

No particular limitation is imposed on the salt of the compunds of the present invention, said salt also pertaining to the present invention, insofar as it is a pharmacologically acceptable salt. Illustrative can be acid addition salts of mineral acids, such as the hydrochloride, hydrobromide, hydroiodide, sulfate, nitrate and phosphate; and acid addition salts of organic acids, such as the benzoate, methanesulfonate, ethane-sulfonate, benzenesulfonate, p-toluenesulfonate, oxalate, maleate, fumarate, tartrate and citrate.

Further, the compounds according to the present invention may exist in the form of solvates represented by hydrates. Further, the compounds according to the present invention may exist as geometric isomers. Such geometric isomers should also be encompassed by the present invention. Further, the compounds according to formulae (I) and (II) are optically active and exist in the form enantiomers. Such enantiomers may be obtained in pure form accdording to conventional resolution methods and should also be encompassed by the present invention.

Some of the compounds are covered by the present claims are commercially available from different sources and have Registry numbers such as [305870-48-8], The compounds according to the present invention can be prepared, for example, by the following processes.

Compounds of general formula (A) may be prepared according to the following scheme (A): 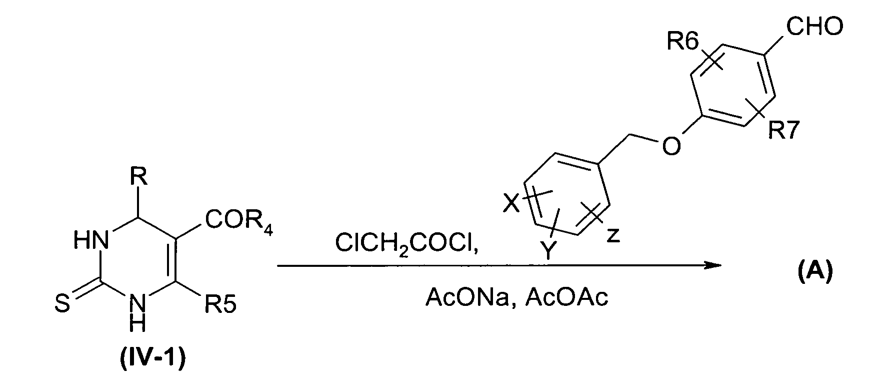 wherein R, R₄ to R₇, X, Y, and Z are as defined above.

Compounds of general formula (A) may be prepared according to the following scheme (B): 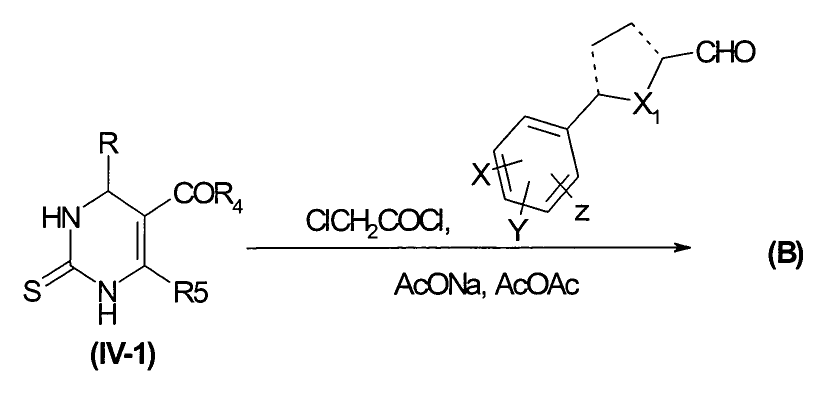 wherein R, R₄ , R₅, X₁, X, Y, Z, and the dotted lines are as defined above.

Compounds of general formula (A) and (I) may be prepared according to the following scheme 1 : 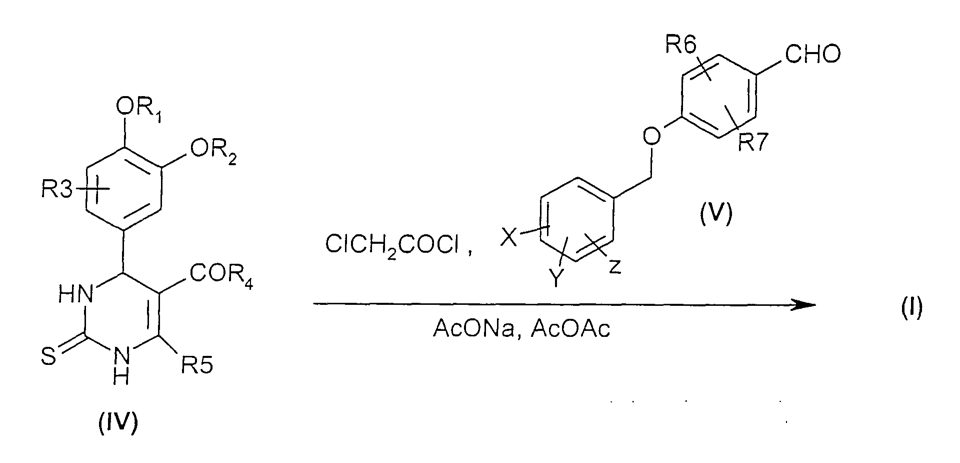 wherein R₁ to R₇, X, Y, and Z are as defined above.

Compounds of general formula (B) and (II) may be prepared according to the following scheme 2:

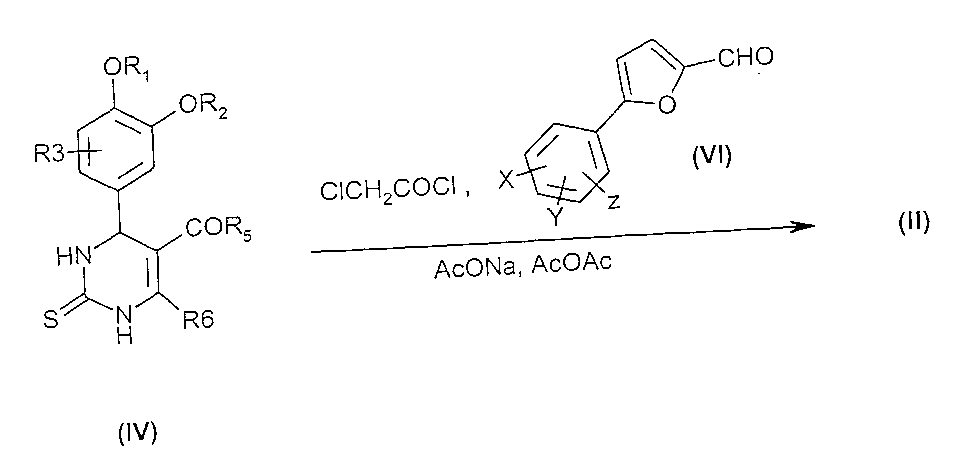

The conversion of starting compound (IV) to the compounds of the invention according to formulae (A), (I) and (B), (II) as shown in reaction scheme(A), (B), 1 and 2 may be carried out according to the procedure disclosed in *Ultra Scientist of Physical Sciences,* 12(3), 277-280 (2000); *Oriental Journal of Chemistry,* 16(3), 427-430, (2000). Accordingly, a compound (IV) is treated with chloroacetyl chloride and a suitably substituted aldehyde (V) or (VI) in acetic anhydride in the presence of a base such as sodium acetate at a temperature of from -30°C to the boiling point of the mixture. A general preparative method is known from Birsen Tozkoparan *et al. , Arch Pharm. Pharm. Med. Chem.* 331, 201-206 (1998).

Starting compounds (IV-1) may be prepared by a Biginelli type reaction according to the following reaction scheme:

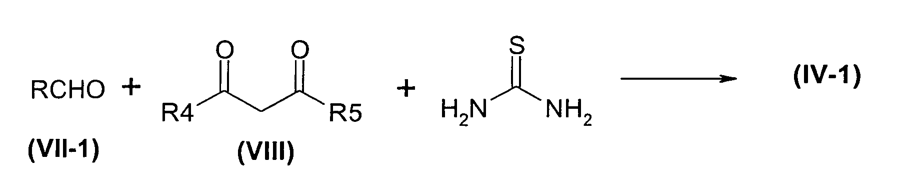

Starting compounds (IV) may be prepared by a Biginelli type reaction according to the following reaction scheme 3: 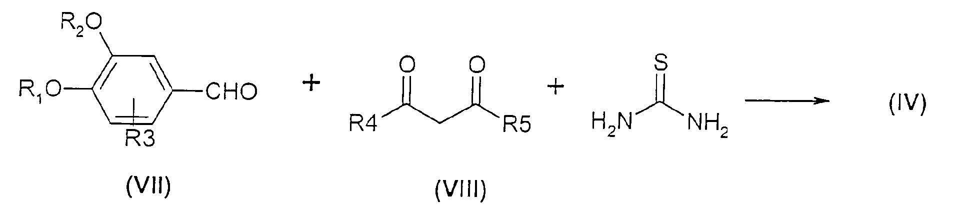 wherein R₁ to R₅, X, Y, and Z are as defined above.

In the preparation of starting compound (IV-1) or (IV), a suitably substituted aldehyde compound (VII-1) or (VII) is reacted with an equimolar amount of a suitably substituted β-keto ester (Villa) or 1,3-diketone (Vlllb) and a slight excess of thiurea in a suitable solvent such as an alcohol at a temperature of from 0°C to the boiling temperature of the reaction mixture. Reference is made to P. Biginelli, *Ber.* **24,** 1317, 2962 (1896); **26** 447 (1893); Martin Zaug, *Organic* Reacts, **14,** 88, (1965); D. J. Brown, *The Pyrimidines,* (Wiley, New York, 1962), p. 440; *ibid.,* Supplement I, **1970**, p. 326; F. Sweet, Y. Fissekis, *J. Am. Chem Soc.,* **95,** 8741 (1973), *Tetrahedron,* **58**, 4801-4807 (2002), J. *Chem. Soc. Perkin Transactions,* **1**, 1845-1846. (2002), and US-A 5,958,931. A general preparative method is known from Mevlüt Ertan *et al., Arch Pharm. (Weinheim)* 324, 135-139 (1991).

Compounds of general formula (C) may be prepared according to the following reaction scheme: 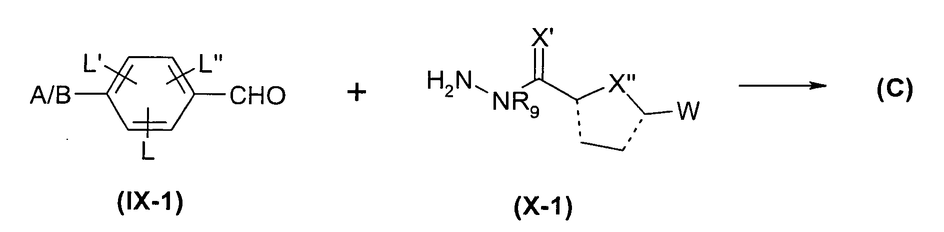 wherein L, L', L", X', X", R_{9'}, W and the dotted lines are as defined above.

Compounds of general formula (C) and (III) may be prepared according to the following reaction scheme 4: 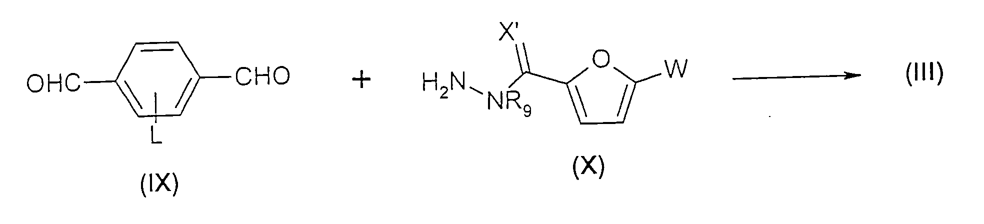 wherein L, X', R₉ and W are as defined above.

Accordingly, a compound of formula (IX) or (IX-1) is condensed with a compound of formula (X) or (X-1), respectively, according to a procedure as disclosed e.g. in *Synthesis,* **5**, 411-413, (1986). Starting material (X) may be prepared by converting 5-nitrofurane 2-carboxylic acid [645-12-5] to the corresponding acid chloride and reaction of the acid chloride with hydrazine as described by Paulsen, Stoy in *The Chemistry of Amides,* Wiley, New York, 1970, page 515-600.

The compounds of formulae (A), (B), (C), (I), (II) and (III) and pharmaceutically acceptable salts or esters thereof can be used as medicaments, e.g. in the form of pharmaceutical preparations.
The pharmaceutical preparations can preferably be administered orally, e.g. in the form of tablets, coated tablets, dragees, hard and soft gelatine capsules, solutions, emulsions or suspensions. However, the administration can also be effected rectally, e.g. in the form of suppositories, or parenterally, e.g. in the form of injection solutions.

The present invention provides a pharmaceutical composition which comprises a compound of formula (A), (B), (C), (I), (II) or (III), in particular a preferred compound as described above, and a pharmaceutically acceptable carrier. The compounds of formula (A), (B), (C), (I), (II) or (III) and pharmaceutically acceptable salts or esters thereof can be processed with pharmaceutically acceptable carriers, e.g. inert, inorganic or organic carriers for the production of pharmaceutical preparations. Lactose, corn starch or derivatives thereof, talc, stearic acid or its salts and the like can be used, for example, as such carriers for tablets, coated tablets, dragees and hard gelatine capsules. Suitable carriers for soft gelatine capsules are, for example, vegetable oils, waxes, fats, semi-solid and liquid polyols and the like; depending on the nature of the active substance no carriers are, however, usually required in the case of soft gelatine capsules. Suitable carriers for the production of solutions and syrups are, for example, water, polyols, sucrose, invert sugar, glucose and the like. Adjuvants, such as alcohols, polyols, glycerol, vegetable oils and the like, can be used for aqueous injection solutions of water-soluble salts of compounds of formula (A), (B), (C), (I), (II) or (III), but as a rule are not necessary. Suitable carriers for suppositories are, for example, natural or hardened oils, waxes, fats, semi-liquid or liquid polyols and the like.

In addition, the pharmaceutical preparations can contain preservatives, solubilizers, stabilizers, wetting agents, emulsifiers, sweeteners, colorants, flavorants, salts for varying the osmotic pressure, buffers, masking agents or antioxidants. They can also contain still other therapeutically valuable substances.

Medicaments containing a compound of formula (A), (B), (C), (I), (II) or (III) or a pharmaceutically acceptable salt or ester thereof and a therapeutically inert excipient are also an object of the present invention, as is a process for the production of such medicaments which comprises bringing one or more compounds of formula (A), (B), (C), (I), (II) or (III) or pharmaceutically acceptable salts or esters thereof and, if desired, one or more other therapeutically valuable substances into a galenical dosage form together with one or more therapeutically inert carriers.

Accordingly, also part of this invention is a method of treating cardiovascular disease such as atherosclerosis whereby the method comprises administering to a patient having any of the above conditions an amount of the pharmaceutical composition of this invention effective to treat or prevent said condition.

The dosage can vary within wide limits and will, of course, be fitted to the individual requirements in each particular case. In general, the effective dosage for oral or parenteral administration is between 0.01-20 mg/kg/day, with a dosage of 0.1-10 mg/kg/day being preferred for all of the indications described. The daily dosage for an adult human being weighing 70 kg accordingly lies between 0.7-1400 mg per day, preferably between 7 and 700 mg per day.

Compounds according to the invention give a positive result as an inhibitor in a cellular and cell-free assay , wherein an IKK-complex is used, which is an immunoprecipitated IKK-complex including an anti-IKK-NEMO antibody. In the cell free assay step (a) preferably comprises subjecting cells to TNFα, followed by isolating the IKK- complex by immunoprecipitation using protein A and an anti-IKK-NEMO antibody. In the cell-free method, test compound is preferably added after step (a). In the cellular assay, compounds are added prior to step (a). In the assay identification is preferably based on an amplified luminescent proximity homogeneous assay and wherein preferably the substrate peptide is biotinylated and immobilized on a streptavidin donor bead. The antibody is preferably immobilized on a protein A-acceptor bead. The substrate peptide of the assays is preferably IkBalpha or Btn-Ahx- GLKKERLLDDRHDSGLDSMKDEE. Preferably, the antibody specific for the IKK-beta phosphorylated domain of the stubstrate peptide is anti-phospho-I kappaB alpha- antibody. Compounds according to the invention are selected from small molecules, preferably non-peptide molecules, having a molecular weight of less than 1500 daltons, more preferably less than 1000 daltons. The compounds are preferably compounds containing an unbranched chain of at least three or four optionally substituted carbocyclic or heterocyclic rings which may be spaced apart by a spacer having a length of not more than four optionally substituted carbon or nitrogen atoms.

### BRIEF DESCRIPTION OF THE DRAWINGS

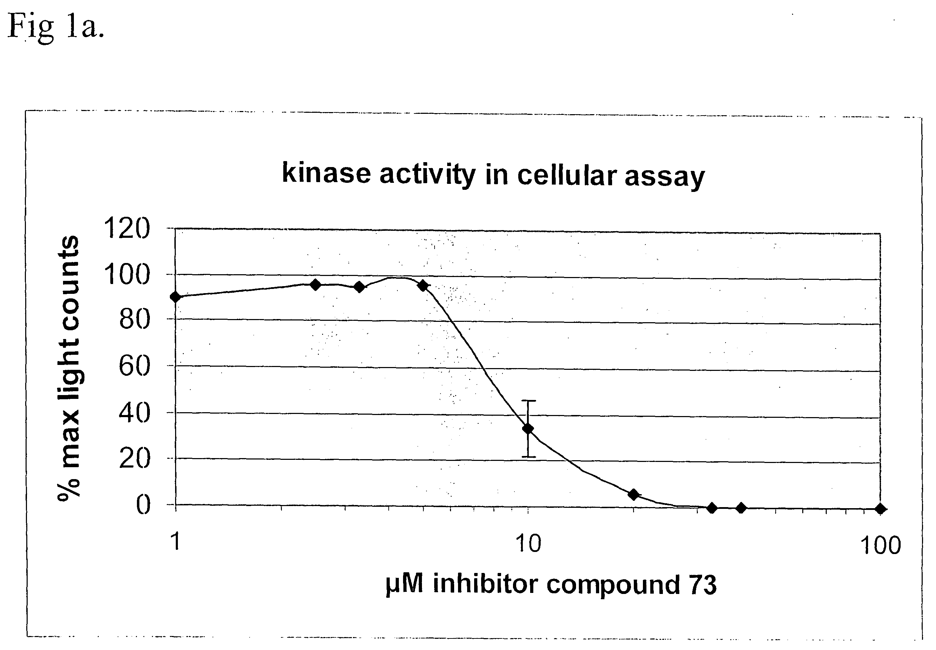

Fig 1a. Effects of compound 73 on activity of IKK-complex after TNF alpha stimulation. The dose-reponse curve of compound 73 for the inhibition of I kappaB peptide phosphorylation is demonstrated. HeLa cells were stimulated with TNF alpha (20 ng/ml) and the activity of the immunoprecipitated IKK-complex was determined in an alpha screen assay (Perkin Elmer). The relative decrese of the activity is determined in % of the maximal fluorescent counts. The summary of n= 3 experiments is shown as means +/- SEM

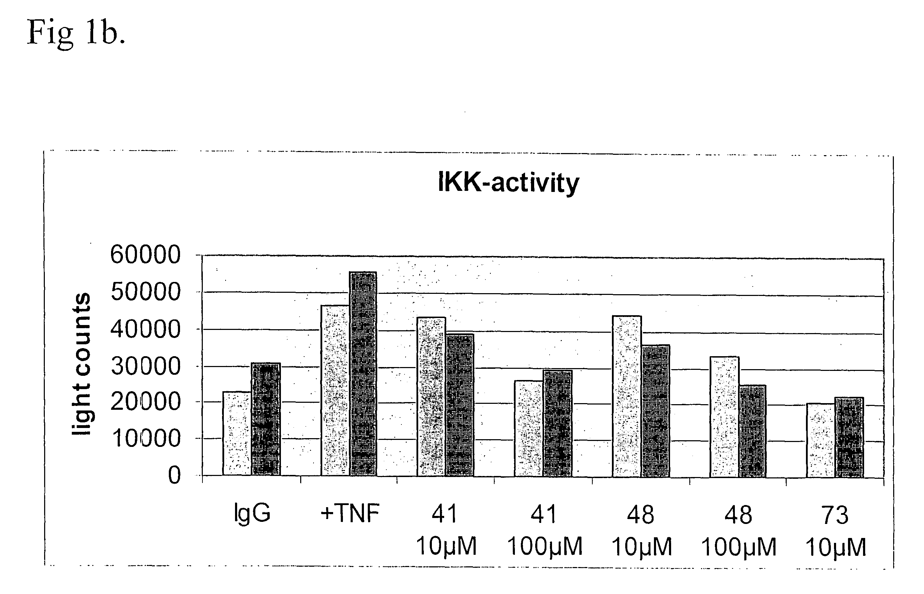

Fig 1 b. Differential inhibition of compound 41, 48 and 73 to the IKK-complex. Double IKK-activity measurements were done after treatment of HeLa cells with various concentrations of the inhibitors (#41; #48 and #73) after TNF alpha (20 ng/ml) stimulation. IKK-complex was immunoprecipitated with anti-NEMO-antibody or in the control with unspecific IgG. The decrease of the kinase activity for I kappaB peptide phosphorylation was determined and fluorescent counts were measured by the alpha screen system (Perkin Elmer).

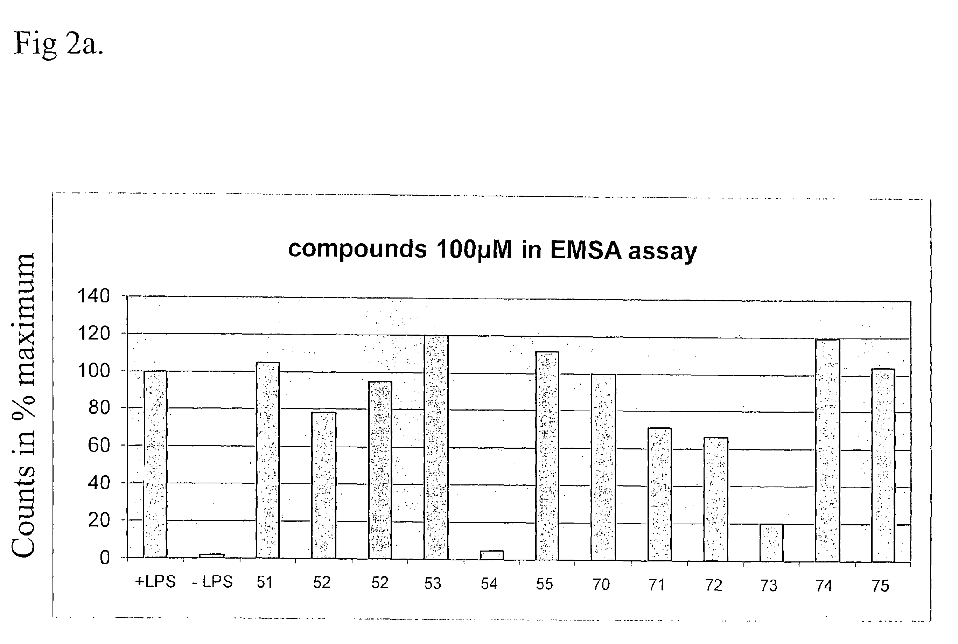

Fig 2a. Effects of different compounds on NF-kappaB activation. THP-1 cells were treated with 100 µM of different compounds for 1 h followed by stimulation with LPS (1 µg/ml). Electrophoretic mobility shift assay (EMSA) for NF-kappaB was carried out. The nuclear extract was incubated with radio-labelled DNA probes with the specific binding sequence for NF kappaB. Signal intensity for labelled NF-kappaB detected by X-ray film exposure was analysed by densitometry. The inhibitory effect on NF-kappaB activity of 10 tested compounds is demonstrated normalized to SP-1 binding relative to 100% of LPS control. Cells without LPS stimulation did not show significant NF-kappaB activity.

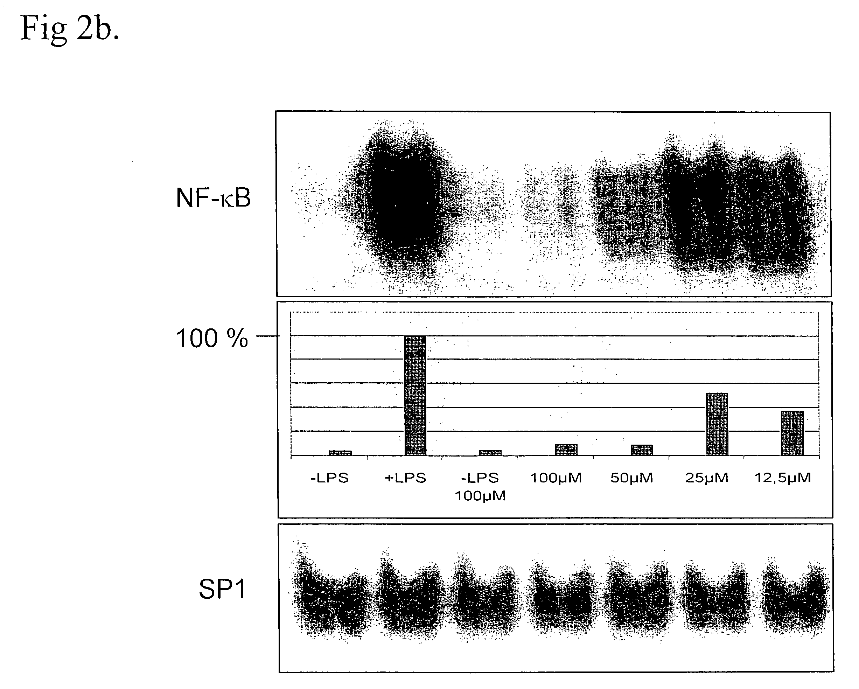

Fig 2b. Dose dependent inhibition of NF-kappaB release with compound 54. Different concentrations of compound 54 were added to THP-1 cells for 1 h. After 1-h stimulation with LPS (1 µg/ml) nuclear extract was prepared for analysing the NF-kappaB activity in the EMSA assay. In the top frame a representative X-ray film after exposure to the EMSA gel is demonstrated. The diagram summarises the dose-dependent inhibition of NF-kappaB activity normalized to SP-1 binding relative to 100% LPS control. In the bottom frame oligonucleotide binding to SP1 is shown as a control.

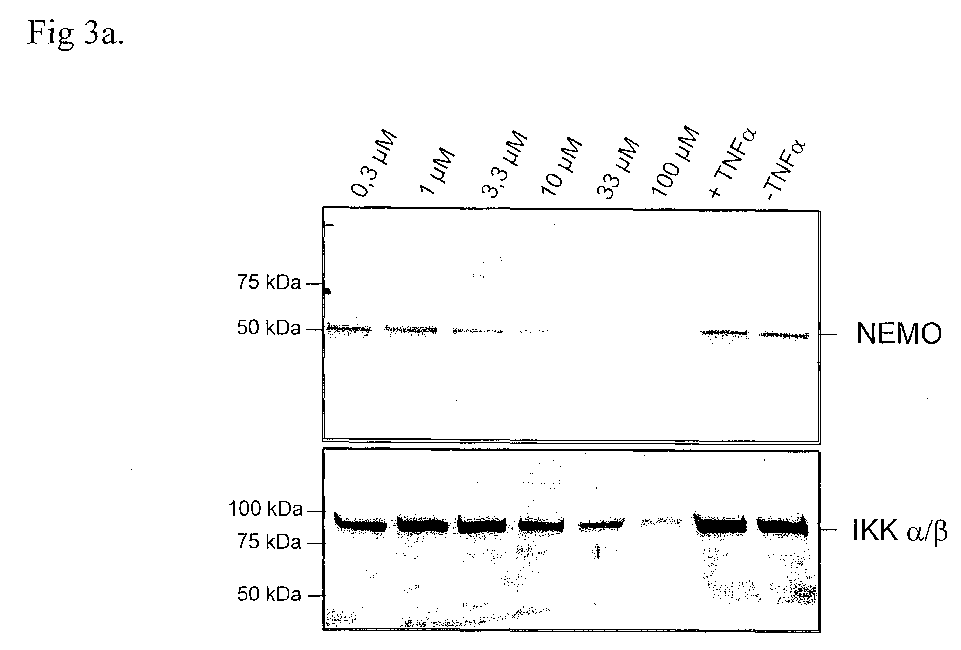

Fig 3a. Degradation of the IKK-complex is triggered by compound 73. HeLa cells were incubated with various concentrations of compound 73 followed by TNF alpha stimulation (20 ng/ml). Additional two controls with or without TNF alpha stimulation were analysed. The amount of NEMO was determined in cell lysates by Western-Blot analysis with a specific anti-NEMO-antibody (Santa Cruz). B. In the same extract the level of IKK alpha/beta was investigated with specific anti-IKK alpha/beta-antibody (Santa Cruz). The representative Westernblots shows dose dependent proteolysis of NEMO and at higher compound 73 concentrations of IKK-alpha and IKK-beta.

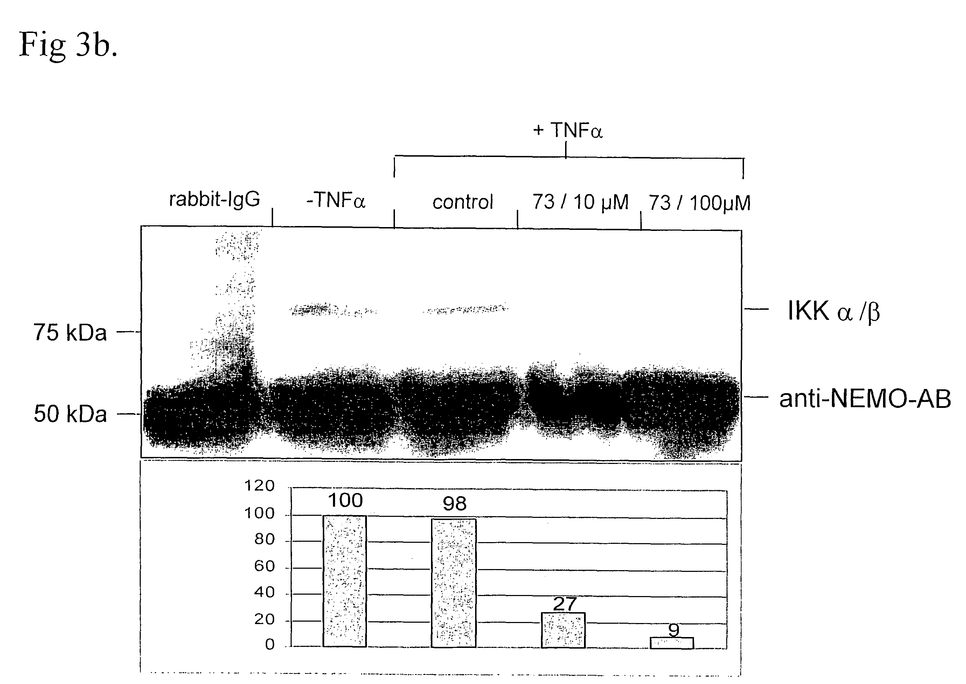

Fig 3b. Disruption of the IKK alpha/beta complex to NEMO binding by compound 73. HeLa cells were treated with 10 µM and 100 µM of compound 73, followed by TNF alpha stimulation (20 ng/ml). IKK-complex was co-precipitated from cytosolic extract with an anti-NEMO-antibody (Santa Cruz) and Protein A-Sepharose. The precipitate was analysed for the IKK alpha/beta protein in Westem-Blot with a specific anti-IKK alpha/beta-antibody. In the control with unspecific rabbit-lgG antibody for immnoprecipitation, no co-precipitation with IKK alpha/betawas detectable. Compound 73 dose-dependently inhibited the binding of IKK alpha/beta to NEMO in activated HeLa cells.

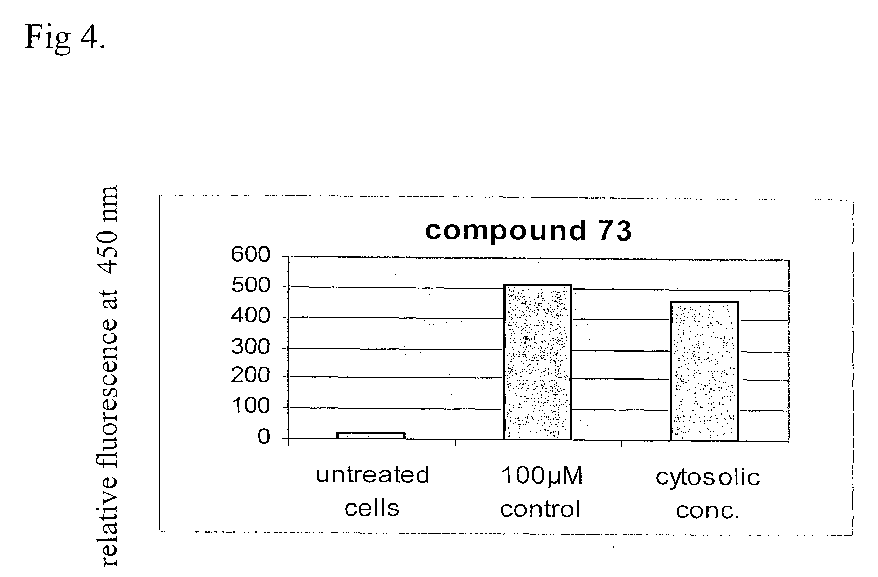

Fig 4. Cell permeability of compound 73. Cytosolic extract was prepared and analysed by Elisa. Compound 73 gives a characteristic signal at 450 nm wavelength in the ELISA reader. Intact HeLa cells were treated 1 h with 100 µM of compound 73. The diagram shows the characteristic signal from the cytosolic extract after incubation with compound 73 in comparison to a 100 µM sample in buffer. Cytosolic extracts from untreated cells are shown in control.

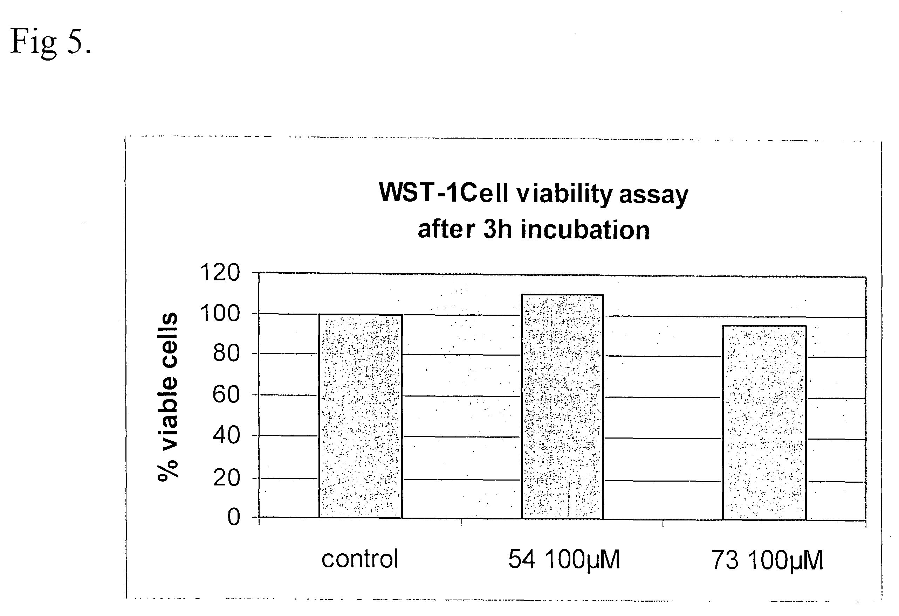

Fig 5. Influence in cell viability of compound 73 and 54. HeLa cells were incubated for 3 h with compound 73 (100 µM) or compound 54 (100 µM). For cell integrity and active metabolism WST-1 reagent was added. The absorbance was determined at the characeristic wavelength with an Elisa reader.

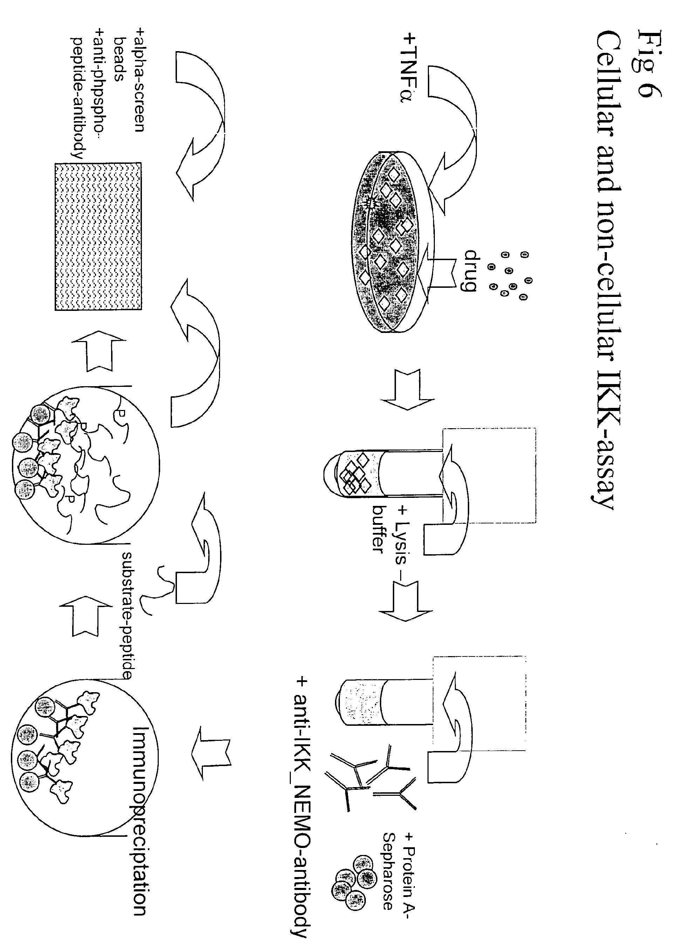

Fig. 6 is a schematic representation of the cellular and non-cellular assays characterising the compounds of the invention by providing positive results as inhibitors.

Fig 7. Chemical structures of the IKK-inhibitors of general formula (B) (COM 73 family). The chemical structures of IKK-inhibitors with structural similarities to the compound COM 73 are shown in comparison. The efficacy of IKK-activity inhibition for these 8 compounds is given in % inhibition of IKK activity measured by NEMO degradation as described in the methods.

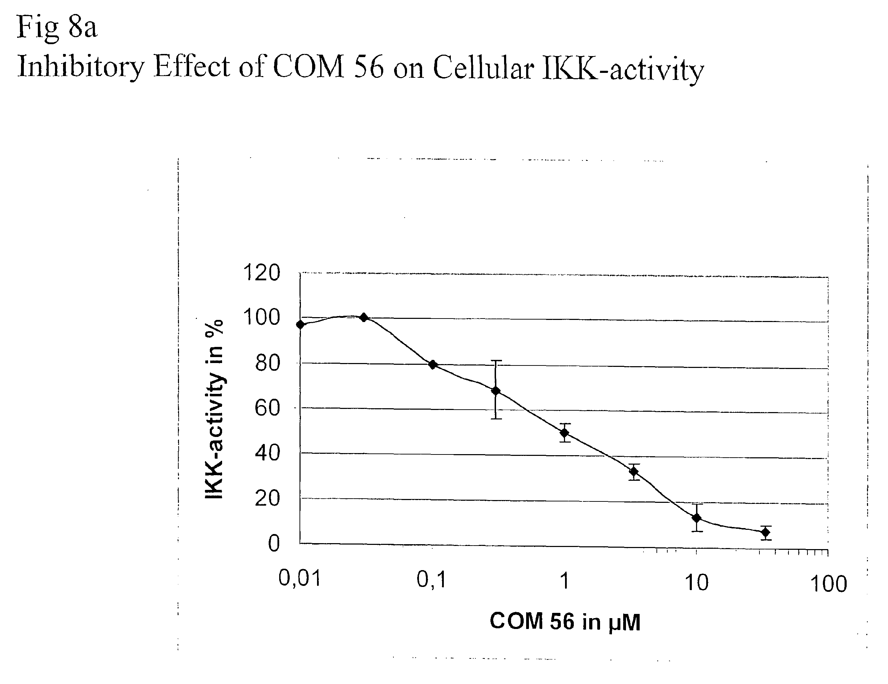

Fig 8a. Inhibitory effect of COM 56 on cellular IKK-activity. The dose-response curve of compound 56 for the inhibition of IKK activity is demonstrated in a cellular assay. In the presence of increasing concentrations of COM 56, HeLa cells were stimulated with TNFα (20 ng/ml). The IKK-complex was immuno-precipitated and the activity of the IKK was determined in an alpha screen assay (Perkin Elmer) as described in the methods. The relative decrease of the activity is determined in % of the maximal fluorescent counts. The summary of n= 4 experiments is shown as means +/- SEM.

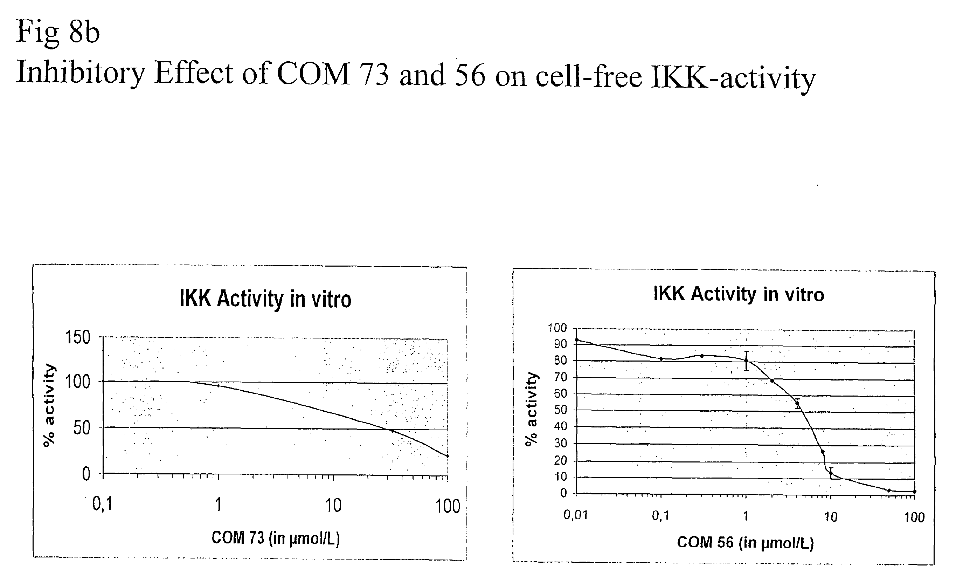

Fig 8b. Inhibitory effect of COM 73 and 56 on cell-free IKK-activity. The dose-response curve of compound 73 and 56 for the inhibition of IKK activity is demonstrated in direct comparison in a cell-free assay. HeLa cells were stimulated with TNFα (20 ng/ml) and the IKK-complex was consecutively immuno-precipitated. In the presence of increasing concentrations of COM 73 and 56, the activity of the IKK was determined under cell free conditions with an alpha screen assay (Perkins Elmer) as described in the methods. The relative decrease of the activity is determined in % of the maximal fluorescent counts. The summary of n = 2 experiments is shown as means +/- SEM.

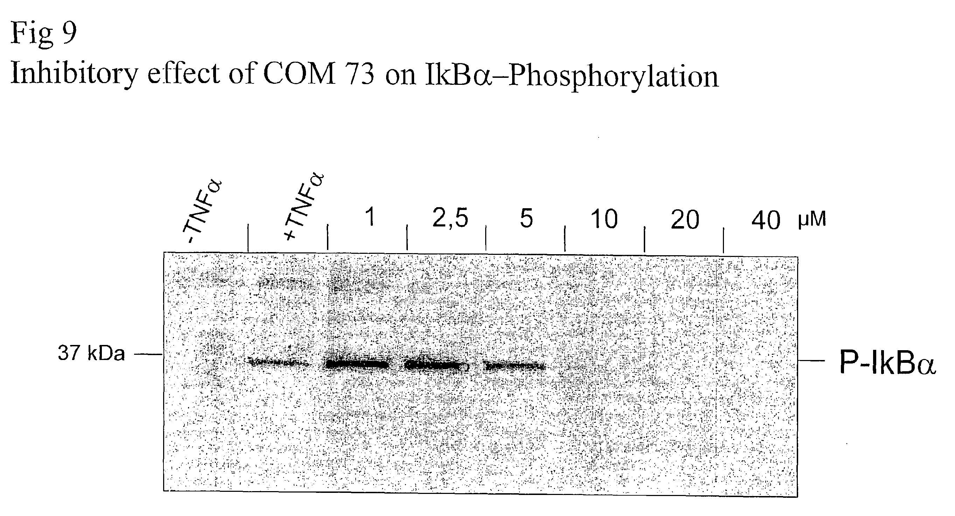

Fig 9. Inhibitory effect of COM 73 on I KappaBalpha phosphorylation. Increasing concentrations of COM 73 does dependently inhibits the phosphorylation of I KappaB alpha. HeLa cells were pre-stimulated with TNFalpha in the presence of increasing concentrations of COM 73. I Kappa B phosphorylation of cell lysates was analysed with specific anti-phosphorylation-antibodies and Western blots. A representative Western blot is demonstrated.

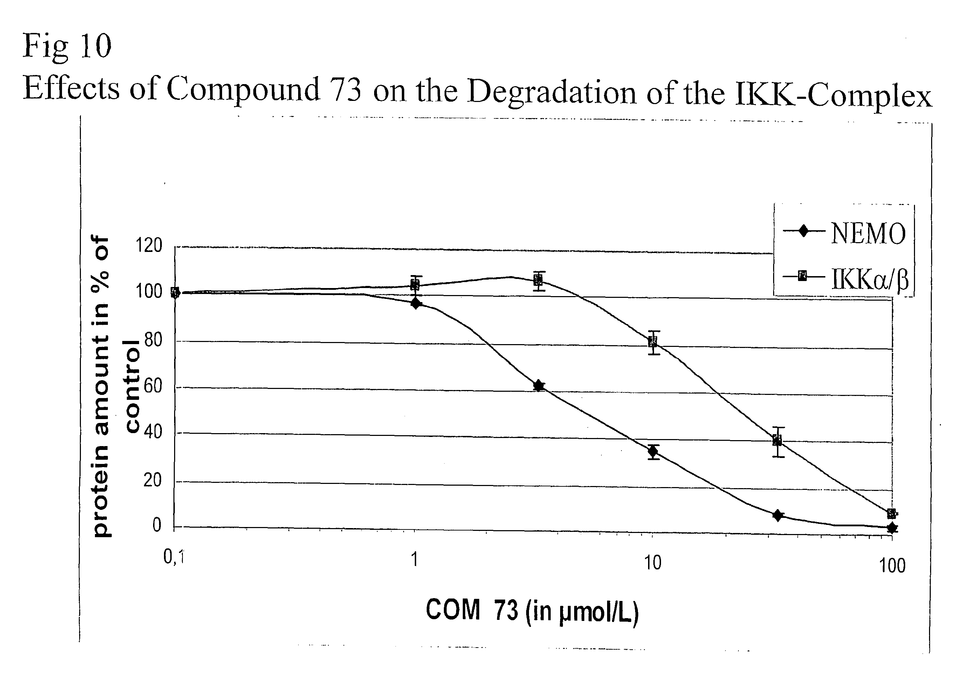

Fig 10. Effects of compound 73 on the degradation of the IKK-complex. The dose-response curve of compound 73 for the degradation of the IKK-complex is demonstrated. HeLa cells were incubated with various concentrations of compound 73 followed by TNFα stimulation (20 ng/ml). After lysis of the cells the amount of NEMO protein was determined by Western-Blot analysis with a specific anti-NEMO-antibody (Santa Cruz). The amount of IKKα/β was investigated with specific anti-IKKa/b-antibody (Santa Cruz) and Western blot. Protein amount was quantified and the relative decrease of the amount of protein by increasing concentrations of COM 73 is determined in % of control . The summary of n = 2 experiments is shown as means +/- SEM.

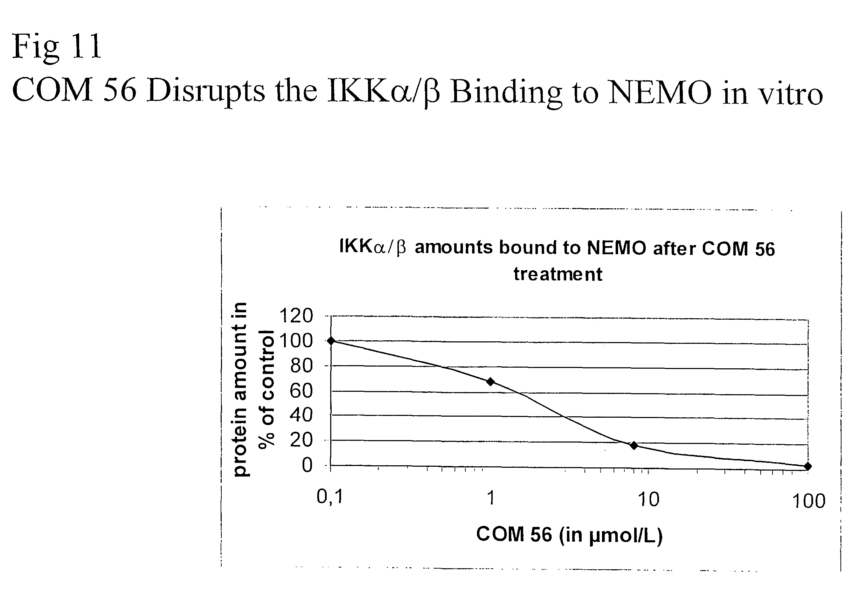

Fig 11. COM 56 disrupts the IKKα/β binding to NEMO in vitro. HeLa cells were treated with TNFα stimulation (20 ng/ml). IKK-complex was co-precipitated from cytosolic extracted with an anti-NEMO-antibody (Santa Cruz) and Protein A-Sepharose. The precipitate was incubated with compound 56 and the complex integrity was analysed for the IKKα/β protein in Western-Blot with a specific antibody. Compound 56 dose-dependently disrupted the binding of IKKα/β to NEMO in vitro. The amount of IKKα/β protein is expressed in % of untreated HeLa cells.

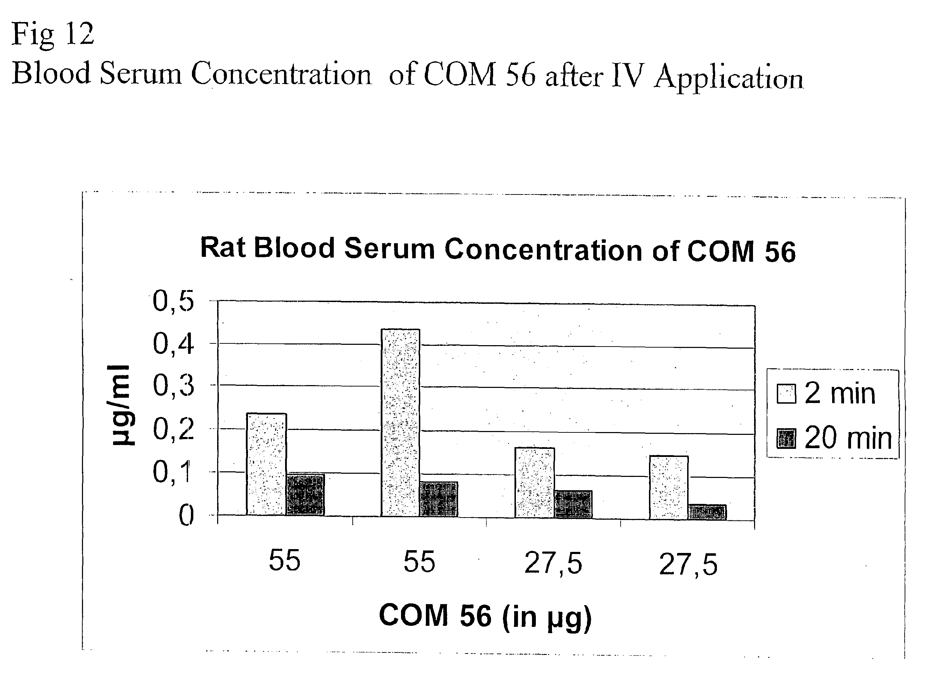

Fig 12. Blood serum concentrations of COM 56 after IV application. The serum levels of COM 56 after single dose IV application was analysed by mass spectrometry. With 27.5 and 55 µg IV injection of COM 56 considerable serum levels could be determined in rats after 2 and 20 minutes. Single values of 2 experiments are given.

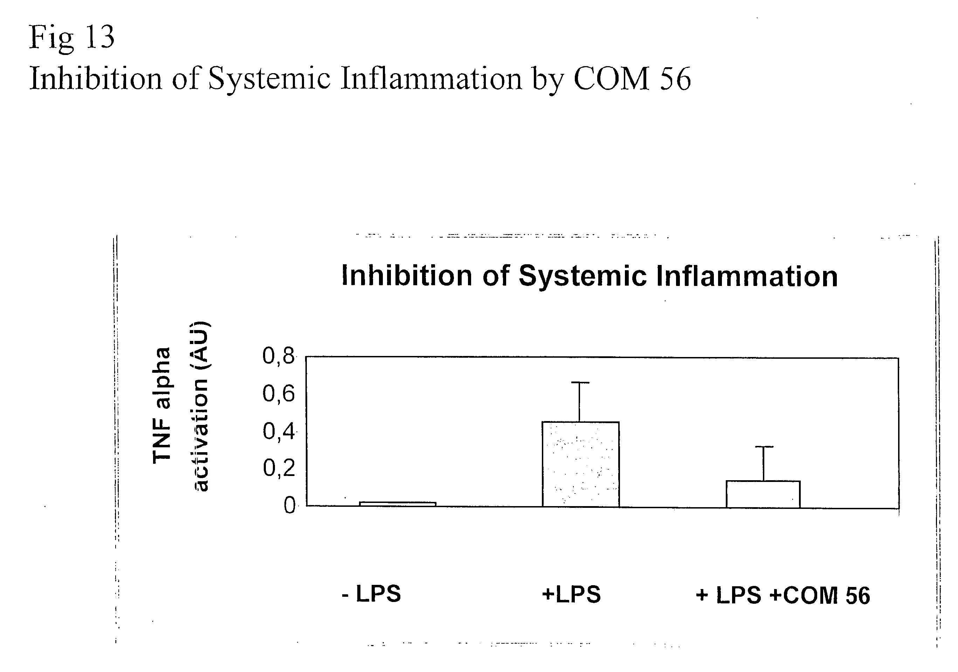

Fig 13. Inhibition of systemic inflammation by COM 56. Systemic TNFalpha release before and after LPS stimulation was determined in mice with a specific ELISA assay. Pre-treatment of mice with COM 56 inhibits systemic TNFalpha release. The means ± SEM of n = 4 experiments is shown.

Fig 14. Chemical structures of the IKK inhibitors of the COM 54 family. The chemical structures of IKK-inhibitors with high structural similarities to the compound COM 54 are shown in comparison.

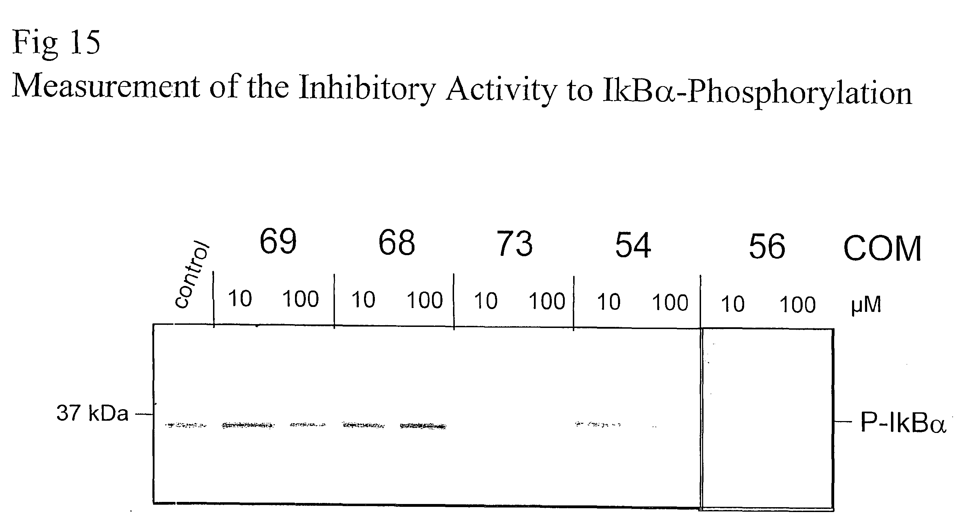

Fig 15. Measurement of the inhibitory activity to IkBα-phosphorylation. The effect of different IKK inhibitors on I KappaB alpha phosphorylation is shown in comparison. HeLa cells were pre-stimulated with TNFalpha in the presence of 10 and 100 µM of different IKK inhibitors. I KappaB phosphorylation of cell-lysates was analysed with specific anti-phosphorylation-antibodies and Western blots. A representative Western blot is demonstrated.

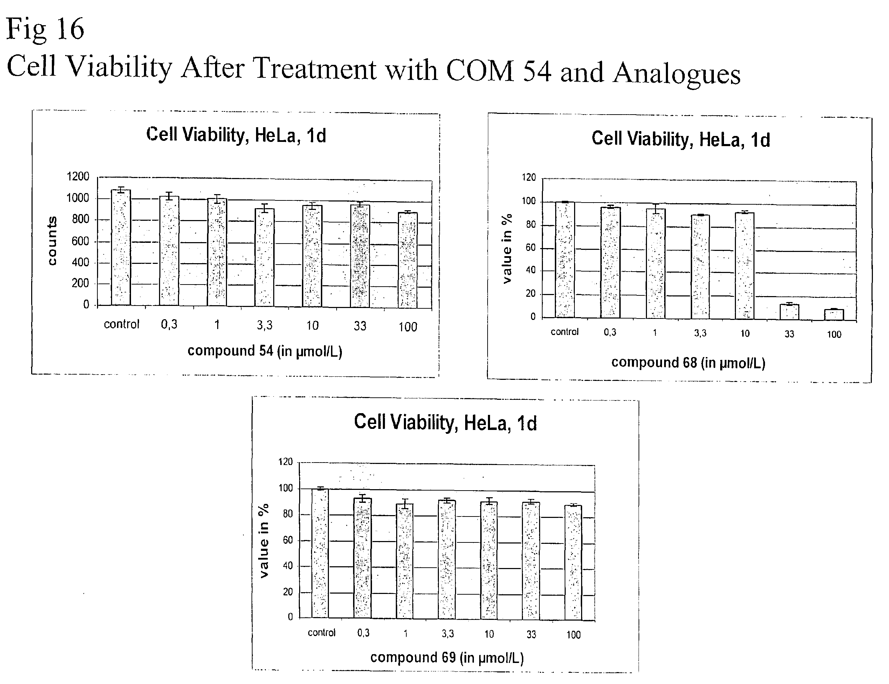

Fig 16. Cell viability after treatment with COM 54 and analogues. HeLa cells were incubated for 1 day with compound 54 and its structural analogues with increasing concentrations. For cell integrity and active metabolism WST-1 reagent was added. The absorbance was determined at the characteristic wavelength with an Elisa reader and determined in % of control. The means ± SEM of n = 4 experiments is shown.

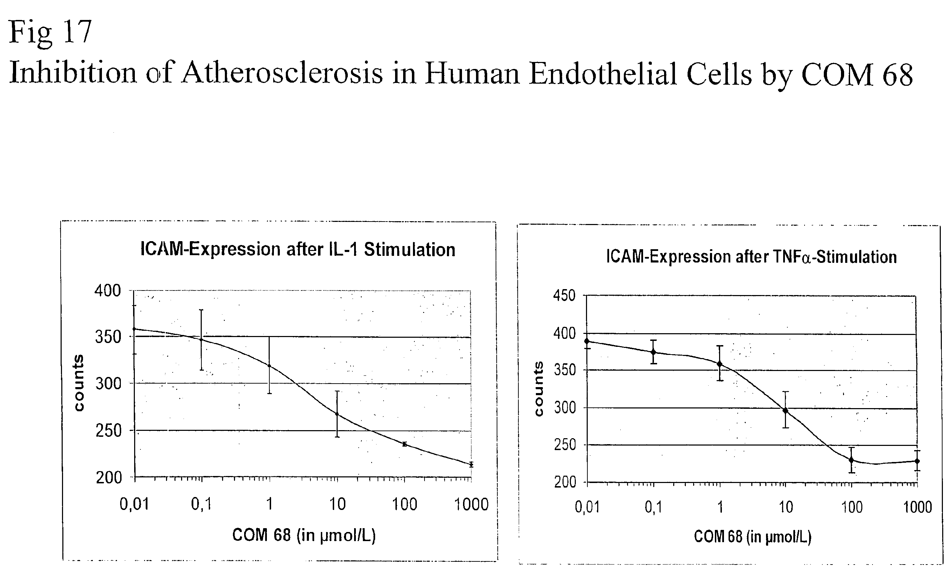

Fig 17. Inhibition of atherosclerosis in human endothelial cells by COM 68. The inhibition of ICAM expression by COM 68 in HUVEC cells is shown both after IL-1 and TNFalpha stimulation.
ICAM expression was determined by FACS measurements of HUVEC cells. The means ± SEM of n = 3 experiments are shown.

The examples which follow are provided by way of illustration and do not limit the invention in any way.

### EXAMPLES

### Example 1: Inhibition of NF-kB dependent I kappaB peptide phosphorylation

### (A) Methods

Kinase Assay Protocol: HeLa cells were maintained in Dulbecco's modified Eagle's medium (Invitrogen) supplemented with 10% fetal bovine serum, 2 mM L-glutamine, penicillin (50 units/ml) and streptomycin (50 µg/ml). 24 h before treatment with different compounds HeLa cells were plated at a density of 5 x 10⁶ per well in 100-mm cell culture dishes to 90% confluences.

The cells were incubated with different drugs at the concentration indicated for 1 h, washed twice with PBS and stimulated with 20 ng/ml TNF alpha (Roche). After 7 min the cells were washed twice with ice cold PBS followed by scraping and transferring into a 1,5 ml microcentrifuge tube. After centrifugation by 2000rpm for 2 min by 4°C the PBS supernatant was removed and 200µl Lyse-buffer (10 mM Hepes, pH 7.9, 0,1 % NP40, 10 mM, 300 mM Sucrose, 10 mM KCI, 15 mM MgCl₂, 1 mM DTT, 0,5 mM PMSF and antipain, aprotinin, leupeptin each 0,75 µg/ml (Sigma) was added to the pellet. The resuspended pellet was incubated on ice for 5 min and centrifuged at 13000 g for 30 s. The supernatant, cytosolic extract, was added to 200 µl TNT-buffer ( 200 mM NaCl, 20 mM Tris/HCl pH 7,5, 1 % Triton X-100). Unspecific binding was blocked by incubation with 3 µg of normal rabbit IgG (Sigma) and 6 mg resuspended and prewashed Protein A Sepharose CI-4B (Pharmacia Biotech) for 30 min by 4°C followed by immunoprecipitation for 1,5 h at 4°C with 2 µg of anti-NEMO-antibody (Santa Cruz Biotechnology) and 6 mg resuspented and prewashed Protein A Sepharose Cl-4B (Parmacia Biotech). After washing three times with TNT buffer and three times with kinase buffer (20 mM HEPES, pH 8.0, 10 mM MgCl₂, 100 µM Na₃VO₄, 20 mM -glycerophosphate, 50 mM NaCl, 2 mM dithiothreitol, 0.5 µM phenylmethylsulfonyl fluoride, antipain, aprotinin, leupeptin 0.75 µg/ml each (Sigma)), the kinase reaction was carried out in 25 µl kinase buffer for 60 min at 30°C in the presence 1 mM ATP (Sigma) and 1 µM of the substrate peptide Btn-Ahx-GLKKERLLDDRHDSGLDSMKDEE-amid (Biosyntan). After centrifugation at 16000 g for 1 min 10 µl supernatant was added to a white 384 proxi-plate (Packard). 6,6 µl of detection-buffer (20mM Hepes pH 7,5, 100 mM NaCl, 1% Tween, 0,1 mM BSA, 50 µg/ml Protein A-Acceptor beads, 250 µg/ml Streptavidin-Donor beads ( both Perkin-Elmer), 4 nM anti-phospho-IKB antibody (Santa Cruz Biotechnology) was dispensed to each well. After incubation for 1,5 h the plate was measured by alpha screen reader (Perkin Elmer).

### Results:

The I kappaB-protein is regulated by cytokine-inducible phosphorylation on Ser-32 and Ser-36. To determine whether several compounds inhibited TNF alpha inducible phosphorylation of the IkB protein, TNF alpha-simulated HeLa cells were treated with these compounds. The specific IkB-Kinase-complex (IKK) was immunoprecipitated with anti-NEMO-antibody and incubated with peptide corresponding to the specific phosphorylation side of IKB. The yield of phosphorylated peptide was analysed by alpha screen (Perkin Elmer). Three different compounds, namely 41, 48 and 73, have an effect on the IKK-activity. Compound 73 was tested in a more detail. Compound 73 inhibited the IKK-activity dose dependently with an IC50 of approximately 8 µM (Fig 1a). Compound 41 and 48 inhibited the IKK-activity with an IC50 value in the range of 10 µM -100 µM. (Fig 1b). Compound 41 is only partly soluble in PBS and actual treating concentration is unknown.

Compound 56, which is a structural analogue of the compound 73 family was further tested in the cellular assay with HeLa cells for IkappaB phosphorylation by the alpha screen reader (Perkin Elmer) as described above. Increasing concentrations of the compound 56 added to HeLa cells dose-dependently inhibited the phosphorylation of the substrate peptide for I KappaB Btn-Ahx-GLKKERLLDDRHDSGLDSMKDEE-amid. The potency of the compound 56 to inhibit the IKK activity was 10 times higher than the other 73 family members and was calculated with IC₅₀ of ∼850 nmol/L **(Fig 8a).**

### (B) Methods:

In vitro / cell free kinase assay protocol:HeLa cells were maintained in Dulbecco's modified Eagle's medium (Invitrogen) supplemented with 10% fetal bovine serum, 2 mM L-glutamine, penicillin (50 units/ml) and streptomycin (50 µg/ml). 24 h before treatment with TNFα HeLa cells were plated at a density of 1 x 10⁷ per well in 175-mm cell culture dishes to 90% confluences.

The cells were stimulated with 20 ng/ml TNFα (Roche) without drugs. After 7 min the cells were washed twice with ice cold PBS followed by scraping and transferring into a 1,5 ml micro centrifuge tube. After centrifugation by 2000 rpm for 2 min by 4°C the PBS supernatant was removed and 400µl Lysis-buffer (10 mM Hepes, pH 7.9, 0,1% NP40, 10 mM, 300 mM Sucrose, 10 mM KCI, 15 mM MgCl₂, 1 mM DTT, 0,5 mM PMSF and antipain, aprotinin, leupeptin each 0,75 µg/ml (Sigma) was added to the pellet. The resuspended pellet was incubated on ice for 5 min and centrifuged at 13000 g for 30 s. The supernatant, cytosolic extract, was added to 400 µl TNT-buffer ( 200 mM NaCl, 20 mM Tris/HCl pH 7,5, 1 % Triton X-100). Unspecific binding was blocked by incubation with 3 µg of normal rabbit IgG (Sigma) and 6 mg resuspended and pre-washed Protein A Sepharose Cl-4B (Pharmacia Biotech) for 30 min by 4°C followed by immuno-precipitation for 1,5 h at 4°C with 4 µg of anti-NEMO-antibody (Santa Cruz Biotechnology) and 12 mg resuspended and pre-washed Protein A Sepharose Cl-4B (Pharmacia Biotech). After washing three times with TNT buffer and three times with kinase buffer (20 mM HEPES, pH 8.0, 10 mM MgCl₂, 100 µM Na₃VO₄, 20 mM -glycerophosphate, 50 mM NaCl, 2 mM dithiothreitol, 0.5 µM phenylmethylsulfonyl fluoride, antipain, aprotinin, leupeptin 0.75 µg/ml each (Sigma)), the Protein A Sepharose pellet was split in four identical aliquots. The supernatant was removed and the kinase reaction was carried out in 25 µl kinase buffer for 60 min at 30°C in the presence 1 mM ATP (Sigma), 1 µM of the substrate peptide Btn-Ahx-GLKKERLLDDRHDSGLDSMKDEE-amid (Biosyntan). Then different concentrations of drugs were added to the cell free components of the isolated components necessary for the Ikappa B phosphorylation. After centrifugation at 16000 g for 1 min 10 µl supernatant was added to a white 384 proxi-plate (Packard). 6,6 µl of detection-buffer (20 mM Hepes pH 7,5, 100 mM NaCl, 1% Tween, 0,1 mM BSA, 50 µg/ml Protein A-Acceptor beads, 250 µg/ml Streptavidin-Donor beads ( both Perkin-Elmer), 4 nM anti-phospho-IKBα-antibody (Santa Cruz Biotechnology) was dispensed to each well. After incubation for 1,5 h the plate was measured by alpha screen reader (Perkin Elmer).

### Results:

The inhibitory effect of COM 73 and COM 56 on IKK activity was compared in a cell-free assay. After TNFalpha stimulation of HeLa cells and immunoprecipitation of the IKK complex, increasing concentrations of drugs were incubated with the isolated IKK complex. COM 73 and 56 dose-dependently inhibited the IKK activity in this cell-free assay. The potency of COM 56 was significantly higher compared to COM 73 in the inhibition of IKK activity. These results confirm the ability of the IKK inhibitory drugs to disrupt the IKK complex after the formation of the kinases complex is completed due to TNFalpha stimulation. Thus COM 73 and 56 are therapies suitable for the treatment of inflammatory diseases such as arteriosclerosis and not only as prophylactics to prevent IKK complex formation **(Fig 8b).**

### (C) Methods:

P-IkBα Detektion in Western-Blot Protocol: HeLa cells were maintained in Dulbecco's modified Eagle's medium (Invitrogen) supplemented with 10% fetal bovine serum, 2 mM L-glutamine, penicillin (50 units/ml) and streptomycin (50 µg/ml). 24 h before treatment with different compounds HeLa cells were plated at a density of 5 x 10⁶ per well in 100-mm cell culture dishes to 90% confluences.

The cells were incubated with different drugs at the concentration indicated for 1 h, washed twice with PBS and stimulated with 20 ng/ml TNFα (Roche). After 7 min the cells were washed twice with ice cold PBS followed by scraping and transferring into a 1,5 ml micro centrifuge tube. After centrifugation by 2000 rpm for 2 min by 4°C the PBS supernatant was removed and 200µl lysis-buffer (10 mM Hepes, pH 7.9, 0,1% NP40, 10 mM, 300 mM Sucrose, 10 mM KCl, 15 mM MgCl₂, 1 mM DTT, 0,5 mM PMSF and antipain, aprotinin, leupeptin each 0,75 µg/ml (Sigma) was added to the pellet. The resuspended pellet was incubated on ice for 5 min and centrifuged at 13000 g for 30 s. 30 µl of the supernatant, cytosolic extract, was diluted with Laemmli-buffer (2 % SDS, 2 % 2-Mercaptoethanol, 0,01 % Bromophenol blue, 8 % Glycerine), heated by 60°C for 10 min and loaded to a 4-20% polyacrylamid gels (BioRad). After electrophoresis the proteins were transferred to a nitrocellulose membrane using the wet blotting technique. First the membrane was blocked with Roti-Block (Roth) and afterwards incubated with monoclonal antibodies against P-IkBalpha (Santa Cruz Biotechnology, used at 1:200 dilution). This incubation was followed by the appropriate horseradish peroxidase-conjugated secondary antibody (Dianova) at 1:10000 dilution. Antibody binding was visualized on x-ray film using the Western blot Chemiluminescent Reagent Detection Kit (Santa Cruz).

### Results:

The ability of compound 73 to inhibit Ikappa B phosphorylation was directly measured by anti-phosphorylation antibodies specific for P-IkappaB alpha and western blot. Increasing concentrations of COM 73 dose-dependently inhibited the phosphorylation of IkappaB **(Fig 9)**.

### Results:

The ability of different compounds of the COM 73 and COM 54 family to inhibit I kappaB phosphorylation was compared. I kappaB phosphorlyation was directly measured by anti-phosphorylation antibodies and Western blot. COM 73 and COM 56 concentration-dependently inhibited the phosphorylation of IkappaB, whereas COM 54; COM 68 and 69 had no effect on IkappaB phosphorylation. Thus the inhibitory effect of this class of NF KappaB-inhibitors is independent of IkappaB phosphorylation **(Fig 15).**

### Example 2: Inhibition of NF-kappaB nucleotide binding activity:

### Methods

Electrophoretic Mobility Shift Assay (EMSA): THP-1 monocytic cells (DSM, Braunschweig, Germany) were maintained in suspension in RPMI 1640 (Glutamax-1, low endotoxin) containing 7% fetal calf serum (FCS) (Myoclone super plus, low endotoxin), 100 units/ml penicillin, and 100 mg/ml streptomycin (Life Technologies, Inc., Eggenstein, Germany). For the experiments, the cells were plated at a density of 3 x 10⁶ per well in 6-well culture dishes. Nuclear extracts were prepared by harvesting cells by centrifugation at 1200 rpm for 7 min at 4°C. The cells were resuspended by adding 1 ml ice cold PBS and transferred into a microcentrifuge tube. After centrifugation at 2000 g for 2 min by 4°C the pellet was lysed in 50 µl buffer A (10 mM Hepes, pH 7.9, 0,1% NP40, 10 mM, 300 mM Sucrose, 10 mM KCI, 15 mM MgCl₂, 1 mM DTT, 0,5 mM PMSF and antipain, aprotinin, leupeptin each 0,75 µg/ml (Sigma)). After 5min incubation on ice and centrifugation at 16000g for 5 sec the pellet was washed with 100 µl buffer A. The nuclear pellet was resuspended with 100 µl buffer B ( 20 mM Hepes, pH 7,9, 100 mM KCI, 100 mM NaCl, 1 mM DTT, 20% Glycerol, 0,5 mM PMSF and antipain, aprotinin, leupeptin each 0,75 µg/ml (Sigma)) and sonicated for 10 sec. The probe was pulse centrifuged at 16000g for 5 sec. The nuclear extract was aliquoted and snap-freeze in liquid N₂. Nuclear extracts (5 mg of protein) were incubated with radiolabeled DNA probes (10 ng; 10⁵ cpm) for 30 min at room temperature in 20 ml of binding buffer (20 mM HEPES, pH 7.9, 50 mM KCI, 1 mM dithiothreitol, 0.5 mM EDTA, 10% glycerol, 1 mg/ml bovine serum albumin, 0.2 % Nonidet P-40, 50 ng of poly(dl-dC)/ ml).The prototypic immunoglobulin k-chain oligonucleotide was used as a probe and labelled by annealing of complementary primers followed by primer extension with the Klenow fragment of DNA polymerase I (Boehringer Mannheim) in the presence of [γ⁻³²P]dCTP (>3,000 Ci/mmol; NEN Life Science Products, Brussels, Belgium) and deoxynucleoside triphosphates (Boehringer Mannheim). Samples were run in 0.253 TBE buffer (10 x TBE is as follows: 890 mM Tris, 890 mM boric acid, 20 mM EDTA, pH 8.0) on nondenaturing 4% polyacrylamide gels. The binding of Sp-1 and AP-1 was also analysed by EMSA using specific consensus oligonucleotides (Promega, Heidelberg, Germany) that were labeled with [γ⁻³²P]ATP (>5,000 Ci/mmol, NEN Life Science Products) and T4 polynucleotide kinase (Boehringer Mannheim). Gels were dried and analysed by autoradiography.

### Results:

The EMSA experiments were performed to examine whether a number of 40 compounds affects the activation of NF-kappaB. THP-1 monocytic cells were preincubated with different substances and then stimulated with LPS. The activation and release of NF- was determined by EMSA. In the same nuclear extracts SP-1, another transcriptional activator factor, was examined for protein binding to oligonucleotides comprising the SP-1 consensus sequence (loading control). In the absence of these compound the expected dramatic activation of NF-kappaB can be observed. By treatment with these compounds at 100 µM two substances showed a significant reduced NF-kappaB-release, in detail for compound 54 by 95% and for compound 73 by 80% (Fig. 2a). Additionally we tested compound 54 with different concentrations ranged from 12,5 µM to 100 µM. The NF-kappaB activation was significantly affected by treatment with compound 54 at 12,5 µM and 25 µM, and nearly completely abolished by 50 µM and 100 µM (see Fig 2b). The binding of SP-1, another transcriptional activator factor serving as control, to the specific oligonucleotide was unchanged.in the same nuclear extracts.

### Example 3: Inhibition of NEMO binding to IKK-beta

### (A) Methods

3 x 10⁶ HeLa cells were incubated with different drugs at increasing concentrations for 1 h, washed twice with PBS and stimulated with 20 ng/ml TNF alpha (Roche). After 7 min the cells were washed twice with ice cold PBS followed by scraping and transferring into a 1,5 ml microcentrifuge tube. (After centrifugation by 2000 rpm for 2 min by 4°C the PBS supernatant was removed and 200 µl Lyse-buffer(10 mM Hepes, pH 7.9, 0,1% NP40, 300 mM Sucrose, 10 mM KCI, 15 mM MgCl₂, 1 mM DTT, 0,5 mM PMSF and antipain, aprotinin, leupeptin each 0,75 µg/ml (Sigma) was added to the pellet. The resuspented pellet was incubated on ice for 5 min and centrifuged at 13000 g for 30s.)
Cytosolic extracts were isolated as described earlier.

30 µL cytosolic extract, approximately extract from 4 x 10⁵ cells, was loaded to a 4-20% polyacrylamid gels (BioRad). After electrophoresis the proteins were transferred to a nitrocellulose membrane using the wet blotting technique. First the membrane was blocked with Roti-Block (Roth) and afterwards incubated with polyclonal antibodies against IKK alpha/beta or NEMO (both Santa Cruz Biotechnology, used at 1:200 dilution). This incubation was followed by the appropriate horseradish peroxidase-conjugated secondary antibody (Dianova) at 1:10000 dilution. Antibody binding was visualized on x-ray film using the Western blot Chemiluminescent Reagent Detection Kit (Santa Cruz).

### Results:

In order to examine whether compound 73 selectivity inhibits the binding of NEMO to IKK alpha/beta resulting in complex instability and degradation, HeLa-cells were treated with various concentration of compound 73 and protein stbility was determined (see Fig 3a). The cytosolic extracts were analysed by Western blot analysis. The levels of NEMO and IKK alpha/beta was dose dependently reduced under these experimental conditions (Fig. 3a). In the case of the NEMO protein a higher susceotability to portein degradation by compound 73 can be oberserved. Decreased amounts of NEMO protein can be observed at 3,3µM, with significant degradation by 20 µM and nearly completely abolition by 33 µM and 100 µM. In contrast IKKa/β degradation was obvious only at higher concentrations 33 µM and 100 µM copound 73. No complete decomposition could be detected even at the highest concentration used. No effect of TNF alphaalpha stimulation on complex composition or complex degradation was observed.

To specifically examine the disruption of the IKK complex we analysed protein expression of IKK-alpha/beta after immunoprecipitation with anti-NEMO-antibody (Santa Cruz Biotechnology) (Fig 3b.). A clear correlation in IKKalpha/beta amount before and after immunoprecipitation was found. With unchanged detectiion of NEMO protein, the level of IKKalpha/beta protein was reduced in a dose dependent manner by compound 73. Compared to the control without compound 73 IKK-alpha/IKK-beta was reduced to 27% at 10 µM and 91 % at 100 µM in the co-precipitated complex with NEMO.

### Results:

The degradation of the IKK complex after TNF alpha stimulation was assessed with Western blots and specific antibodies against IKK-alpha/beta and NEMO. COM 73 concentration-dependently degraded IKKalpha/beta and NEMO after incubation with the intact cells. NEMO was more sensitive to protein degradation by COM 73 compared to the IKK alpha/beta complex (Fig 10).

### (B) Methods:

Degradation of the IKK-complex after drug treatment in vitro:
After immuno-precipitation with anti-NEMO antibody (Santa Cruz) as described earlier, the precipitated and washed IKK-complex was incubated with different concentrations of IKK inhibitors in 20 mM HEPES, pH 8.0, 10 mM MgCl₂, 100 µM Na₃VO₄, 20 mM - glycerophosphate, 50 mM NaCl, 2 mM dithiothreitol, 0.5 µM phenylmethylsulfonyl fluoride, antipain, aprotinin, leupeptin 0.75 µg/ml each (Sigma). After 1 h treatment the probe was centrifuged at 16000 g for 1 min the supernatant was removed totally and the protein A pellet was resuspended in 1 x Laemmli-buffer buffer (2 % SDS, 2 % 2-Mercaptoethanol, 0,01 % Bromophenol blue, 8 % Glycerine), heated by 60°C for 10 min and loaded to a 4-20% polyacrylamid gels (BioRad). After electrophoresis the proteins were transferred to a nitrocellulose membrane using the wet blotting technique. First the membrane was blocked with Roti-Block (Roth) and afterwards incubated with monoclonal antibodies against IKKα (Santa Cruz Biotechnology, used at 1:200 dilution). This incubation was followed by the appropriate horseradish peroxidase-conjugated secondary antibody (Dianova) at 1:10000 dilution. Antibody binding was visualized on x-ray film using the Western blot Chemiluminescent Reagent Detection Kit (Santa Cruz). The x-ray film was scanned and the results were analysed by densitometry by the software AIDA.

### Results

Inhibition of NEMO binding to IKK-alpha/beta by compound 56 was assessed after immuno-precipitation of the IKK complex. COM 56 dose-dependently disrupted the complex in this in vitro assay **(Fig 11).**

### Example 4: Cell permeability of NF-kappaB inhibitors

### Methods:

3 x 10⁶ HeLa cells were incubated with compound 73 at 100 µM for 1 h. After washing with PBS three times 120 µl hypotonic buffer (10 mM NaCl, 10 mM Hepes ph 7,5) was added to the cell pellet and frozen in liquid N₂ for cell lysis. After centrifugation at 16000g for 5 min the supernatant was measured by 450 nm in Elisa and amount of compound 73 was compared with a standard concentration of this substances.

### Results:

Cell permeability of compound 73 was monitored by measurement of the compound concentration in the cytoplasm by its characteristic signal in an Elisa reader. After incubation for 1 h with 100 µM of compound 73 high levels of the compoud could be detected. Compared to the signal of the of 100 mM compound a concentration of approximately 90 µM was founded inside the cells (Fig 4.). This result indicates an excellent cell permeability of compound 73.

### Example 5: Cell viability after treatment with NF-kappaB inhibitors

### Methods:

3 x 10⁴ HeLa cells in 96 well plate were incubated with 100 µM of compound 54 and 73 for 3 h. The medium was changed and 10 µL of the WST-1 reagent was added to each well. After 2 h the absorption of 450 nm was measured in an Elisa-reader.

### Results:

A potential toxicity of the compound 73 und 54 was monitored by the WST-1 viability test assay (Boehringer Mannheim). Compound 54 und 73 were not found to be toxic for HeLA cells at the concentrations and conditions applied in the described assays. After incubation with 30 µM of each compound for 3 h no significant decrease in metabolic activity could be detected compared with the untreated control.

### Results:

Cell viability for the compounds of the COM 54 family was tested in HeLa cells. Incubation of HeLa cells with increasing concentrations of COM 54 and 69 (0.3 to 100 µmol/L) for one day had no negative influence on cell viability (assessed by WST staining). COM 68 in concentrations of 33 and 100 µmol/L decreased cell viability after the one day incubation period (Fig 16).

### Example 6: Kinase Assay Protocol

HeLa cells were maintained in Dulbecco's modified Eagle's medium (Invitrogen) supplemented with 10% fetal bovine serum, 2 mM L-glutamine, penicillin (50 units/ml) and streptomycin (50 µg/ml).24 h before treatment with different compounds Hela cells were plated at a density of 5 x 10⁶ per well in 100-mm cell culture dishes to 90 % confluency.

The cells were incubated with different drugs at the concentration indicated for 1 h, washed twice with PBS and stimulated with 20 ng/ml TNFalpha (Roche). After 7 min the calls were washed twice with icecold PBS followed by scraping and transferring into a 1,5 ml microcentrifuge tube. After centrifugation by 2000rpm for 2 min by 4°C the PBS supernatant was removed and 200 *µ*l Lyse-buffer (10 mM Hepes, pH 7.9, 0,1 % NP40, 10 mM, 3 00 mM Sucrose, 10 mM KCI, 15 mM MgCl², 1 mM DTT, 0,5 mM PMSF and antipain, aprotinin, leupeptin each 0,75 *µ*g/ml (Sigma) was added to the pellet. The resuspented pellet was incubated on ice for 5 min and centrifuged at 13000 g for 30 s. The supernatant, cytosolic extract, was added to 200 *µ*l TNT-buffer ( 200 mM NaCl, 20 mM Tris/HCl pH 7,5,1 % Triton X-100). Unspecific binding was blocked by incubation with 3 µg of normal rabbit IgG (Sigma) and 6 mg resuspended and prewashed Protein A Sepharose CI-4B (Pharmacia Biotech) for 30 min by 4°C followed by immunoprecipitation for 1,5 h at 4°C with 2 µg of anti-NEMO-antibody (Santa Cruz Biotechnology) and 6 mg resuspented and prewashed Protein A Sepharose,Cl-4B (Parmacia Biotech). After washing three times with TNT buffer and three times with kinase buffer (20 mM HEPES, pH 8.0, 10 mM MgCl₂, 100 µM Na₃VO₄, 20 mM -glycerophosphate, 50 mM NaCl, 2 mM dithiothreitol, 0.5 µM phenylmethylsulfonyl fluoride, antipain, aprotinin, leupeptin 0.75 µg/ml each (Sigma)), the kinase reaction was carried out in 25 µl kinase buffer for 60 min at 30°C in the presence I mM ATP (Sigma) and I µM of the substrate peptide Btn-Ahx-GLKKERLLDDRHDSGLDSMKDEE-amid (Biosyntan). After centrifugation at 16000 g for 1 min 10 µl Supernatant was added to a white 384 proxi-plate (Packard). 6,6 µl of detection-buffer (20mM Hepes pH 7,5, 100 mM NaCl, 1% Tween, 0,1 mM BSA, 50 µg/ml Protein A-Acceptor beads, 250 µg/ml Streptavidin-Donor beads (both Perkin-Elmer), 4 nM anti-phospho-IKBα-antibody (Santa Cruz Biotechnology) was dispensed to each well. After incubation for 1,5 h the plate was measured by alpha screen reader (Perkin Elmer).

### Example 7: Electrophoretic Mobility Shift Assay (EMSA)

THP- I monocytic cells (DSM, Braunschweig, Germany) were maintained in suspension in RPMI 1640 (Glutamax-1. low endotoxin) containing 7% fetal calf serum (FCS) (Myoclone super plus, low endotoxin), 100 units/ml penicillin, and 100 mg/ml streptomycin (Life Technologies, Inc., Eggenstein, Germany) as described (41). For the experiments, the cells were plated at a density of 3 x 10⁶ per well in 6-well culture dishes. Nuclear extracts were prepared by harvesting cells by centrifugation at 1200 rpm for 7 min at 4°C. The cells were resuspended by adding 1 ml icecold PBS and transferred into a microcentrifuge tube. After centrifugation at 2000g for 2 min by 4°C the pellet was lysed in 50 µl buffer A (10 mM Hepes, pH 7.9, 0,1 % NP40, 10 mM, 300 mM Sucrose, 10 mM KC1, 15 mM MgCl, 1 mM DTT, 0,5 mM PMSF and antipain, aprotinin, leupeptin each 0,75 µg/ml (Sigma)). After 5 min in incubation on ice and centrifugation at 16000g for 5 sec the pellet was washed with 100 µl buffer A. The nuclear pellet was resuspended with 100 µl buffer B ( 20 mM Hepes, pH 7,9, 100 mM KCI, 100 mM NaCl, 1 mM DTT, 20 % Glycerol, 0,5 mM PMSF and antipain, aprotinin, leupeptin each 0,75 µg/ml (Sigma)) and sonicated for 10 sec. The probe was pulse centrifuged at 16000g for 5 sec. The nuclear extract was aliquted and snap-freezed in liquid nitrogen. Nuclear extracts (5 mg of protein) were incubated with radiolabeled DNA probes (;10 ng; 10 5 cpm) for 30 min at room temperatuie in 20 ml of binding buffer (20 mM HEPFS, pH 7.9, 50 mM KCI, 1 mM dithiothreitol, 0.5 mM EDTA, 10% glycerol, 1 mg/ml bovine serum albumin, 0.2% Nonidet P-40, 50 ng of poly(dl-dC)/ ml). The prototypic immunoglobulin k-chain oligonucleotide was used as a probe and labeled by annealing of complementary primers followed by primer ex-tension with the Klenow fragment of DNA polymerase I (Bochringer Mannheim) in the presence of [a-32 P]dCTP (3,000 Ci/mmol; NEN Life Science Products, Brussels, Belgium) and deoxynucleoside triphos-phates (Bochringer Mannheim). Samples were run in 0.253 TBE buffer (103 TBE is as follows: 890 mM Tris, 890 mM boric acid, 20 mM EDTA, pH 8.0) on nondenaturing 4% polyacrylamide gels. The binding of Sp-1 and AP- I was also analyzed by EMSA using specific consensus oligonucleotides (Promega, Heidelberg, Germany) that were labeled with [g-32P] ATP (5,000 Ci/mmol, NEN Life Science Products) and T4 polynucleotide kinase (Boehringer Mannheim). Gels were dried and analyzed by autoradiography.

### Example 8: Pharmacokinetics of IKK inhibitory drugs after single dose IV administration:

### Method

### In vivo IV application in rats:

COM 56 was diluted in saline-buffer to 100 µM and 200 µM in a volume of 200 µL. The probe was administered intravenously in rats. After 2 min and 20 min a blood probe was taken from the right carotid artery. The blood probes were centrifuged by 2500 g for 3 min and the supernatant, the serum component, were analysed by mass spectroscopy.

### Results:

After single IV application of 55 and 27.5 µg compound 56 in rats positive serum probes were measured 2 minutes and 20 minutes after administration **(Fig 12)**.

### Example 9: Inhibition of systemic inflammation

### Methods:

Systemic inflammatory response in mice was induced by lipopolysaccharide (LPS) shock (IV administration of LPS 0.33 µg/g). Inflammatory and inhibition of inflammation by IV administration of compound 56 (1 µg/g) to mice was determined by TNFalpha quantification in mice serum. TNFalpha was detected with an ELISA kit (Pierce) according to the manufacturer's instructions.

### Results:

Systemic inflammation induced by LPS in mice in vivo was inhibited by compound 56. TNFalpha concentrations in mouse serum were significantly inhibited after compound 56 pre-treatment **(Fig 13)**.

### Example 10: Inhibition of atherosclerosis in human endothelial cells by drug treatment

### Methods:

Determination of ICAM expression by fluorescent activated cell sorter (FACS) analysis: HUVEC cells were seeded in 6-well plates 1 x 10⁶ cells/well. After 24h the cells were treated with different concentration of compound 68. After 1 h the medium was removed and the cells incubated with either IL-1 (100 pg/ml) or TNFα (1 ng/ml) for a total of 4 h. Thereafter the cells were harvested with trypsin, washed with PBS and centrifuged with 1000 rpm for 5 min. The pellet was resuspended in 50µl PBS and 5µl CD54-PE antibody ( anti-ICAM antibody from Beckman/ Coulter/ Immunotech). After washing twice in PBS, the ICAM cell surface expression was analysed by a FACScan (Becton Dickinson).

### Results:

COM 68 concentration-dependently inhibited atherosclerosis markers in human endothelial cells. Incubation of COM 68 with HUVEC cells significantly inhibited ICAM expression. After IL-1 stimulation the IC50 for COM 68 was 7.8 µmol/L and after TNFalpha stimulation 4.5 µmol/L for inhibition of ICAM expression **(Fig 17).**

### FORMULATION EXAMPLE A

Tablets of the following composition are produced in a conventional manner:

| | mg/Tablet |
|---|---|
| Active ingredient | 100 |
| Powdered. lactose | 95 |
| White corn starch | 35 |
| Polyvinylpyrrolidone | 8 |
| Na carboxymethylstarch | 10 |
| Magnesium stearate | 2 |
| Tablet weight | 250 |

### FORMULATION EXAMPLE B

Tablets of the following composition are produced in a conventional manner:

| | mg/Tablet |
|---|---|
| Active ingredient | 200 |
| Powdered. lactose | 100 |
| White corn starch | 64 |
| Polyvinylpyrrolidone | 12 |
| Na carboxymethylstarch | 20 |
| Magnesium stearate | 4 |
| Tablet weight | 400 |

### FORMULATION EXAMPLE C

Capsules of the following composition are produced:

| | mg/Capsule |
|---|---|
| Active ingredient | 50 |
| Crystalline. lactose | 60 |
| Microcrystalline cellulose | 34 |
| Talc | 5 |
| Magnesium stearate | 1 |
| Capsule fill weight | 150 |

The active ingredient having a suitable particle size, the crystalline lactose and the microcrystalline cellulose are homogeneously mixed with one another, sieved and thereafter talc and magnesium stearate are admixed. The final mixture is filled into hard gelatine capsules of suitable size.

## Claims

1. A 5H-Thiazolo[3,2]pyrimidine derivative or a salt thereof for use as a medicine, which is represented by the following formula (A): 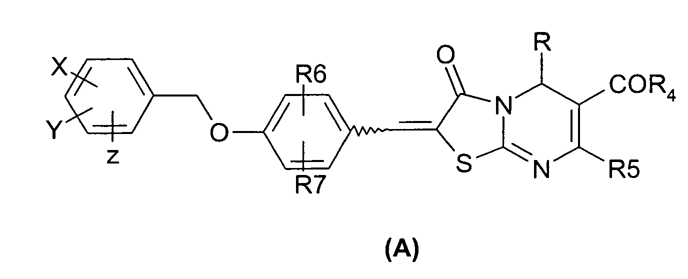 wherein
R represents an optionally substituted phenyl or pyridyl group;
R₄ represents
a hydroxyl group,
an amino group,
a straight chain or branched alkoxy group,
a straight chain or branched alkyl group,
a cycloalkyloxy group,
an alkylamino group,
a cycloalkylamino group,
a dialkylamino group,
R₅ represents
a hydrogen atom,
a straight chain or branched alkyl group,
R₆ and R₇ which may be the same or different represent
a hydrogen atom,
a halogen atom,
a straight chain or branched alkyl group,
a straight chain or branched alkoxy group,
a straight chain or branched alkenyl group,
a cycloalkyl group,
an aryl group,
X, Y, and Z which may be same or different represent
a hydrogen atom,
a halogen atom,
a carboxyl group
a nitro group,
a cyano group,
an alkyl group,
an alkoxy group or
an acyl group.

2. The 5H-Thiazolo[3,2]pyrimidine derivative or a salt or ester thereof for use as a medicine according to claim 1 wherein the phenyl or pyridyl group represented by R is substituted by 1 to 3 substituents selected from the group consisting of a halogens such as fluorine, chlorine, bromine and iodine, a cyano group, a hydroxy group, a nitro group, a carboxyl group, an amino group, a straight chain or branched C₁₋₆ alkyl group, a straight chain or branched C₁₋₆ alkoxy group, a straight chain or branched C₁₋₇ alkylcarbonyl group, a straight chain or branched C₁₋₇ alkoxycarbonyl group, straight chain or branched C₁₋₇ alkoxycarbonyloxy group, a straight chain or branched C₁₋₆alkylamino group, a straight chain or branched di-C₁₋₆alkylamino group, a straight chain or branched C₁₋₇ alkylcarbonylamino group, a straight chain or branched C₁₋₆alkylaminocarbonyl group, a straight chain or branched C₁₋₇ alkoxycarbonylamino group, a straight chain or branched C₁₋₇ alkylaminocarbonyloxy group.

3. The 5H-Thiazolo[3,2]pyrimidine derivative or a salt or ester thereof for use as a medicine according to claim 10, wherein R is an optionally substituted phenyl group.

4. The 5H-Thiazolo[3,2]pyrimidine derivative or a salt or ester thereof for use as a medicine according to claim 10 or 11, wherein 1 to 3 substituents a present which are selected from a halogen atom or an alkoxy group.

5. A 5H-Thiazolo[3,2]pyrimidine derivative or a salt thereof for use as a medicine, which is represented by the following formula (B): 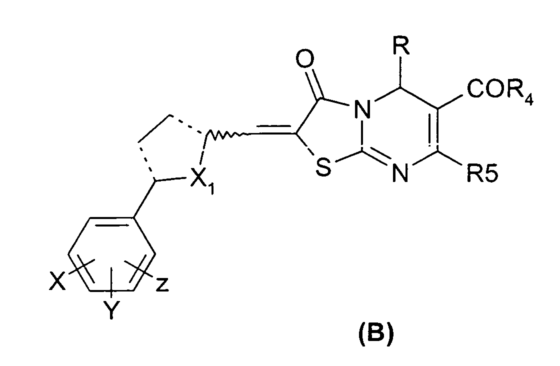 wherein
the dotted lines independently represent a single bond or a double bond,
R represents an optionally substituted phenyl or pyridyl group
R₄ represents
a hydroxyl group,
an amino group,
a straight chain or branched alkoxy group,
a straight chain or branched alkyl group,
a cycloalkyloxy group,
an alkylamino group,
a cycloalkylamino group,
a dialkylamino group;
R₅ represents
a hydrogen atom,
a straight chain or branched alkyl group,
X₁ represents O, S, or NH;
X, Y, and Z which may be same or different represent
a hydrogen atom,
a halogen atom,
a carboxyl group
a nitro group,
a cyano group,
an alkyl group,
an alkoxy group or
an acyl group.

6. The 5H-Thiazolo[3,2]pyrimidine derivative or a salt or ester thereof for use as a medicine according to claim 5 wherein the phenyl or pyridyl group represented by R is substituted by 1 to 3 substituents selected from the group consisting of a halogens such as fluorine, chlorine, bromine and iodine, a cyano group, a hydroxy group, a nitro group, a carboxyl group, an amino group, a straight chain or branched C₁₋₆ alkyl group, a straight chain or branched C₁₋₆ alkoxy group, a straight chain or branched C₁₋₇ alkylcarbonyl group, a straight chain or branched C₁₋₇ alkoxycarbonyl group, straight chain or branched C₁₋₇ alkoxycarbonyloxy group, a straight chain or branched C₁₋₆alkylamino group, a straight chain or branched di-C₁₋₆alkylamino group, a straight chain or branched C₁₋₇ alkylcarbonylamino group, a straight chain or branched C₁₋₆alkylaminocarbonyl group, a straight chain or branched C₁₋₇ alkoxycarbonylamino group, a straight chain or branched C₁₋₇ alkylaminocarbonyloxy group.

7. The 5H-Thiazolo[3,2]pyrimidine derivative or a salt or ester thereof for use as a medicine according to claim 5, wherein R is an optionally substituted phenyl group.

8. The 5H-Thiazolo[3,2]pyrimidine derivative or a salt or ester thereof for use as a medicine according to claim 20 or 21, wherein 1 to 3 substituents a present which are selected from a halogen atom or an alkoxy group.

9. A compound is represented by the following formula (C) or a salt thereof: 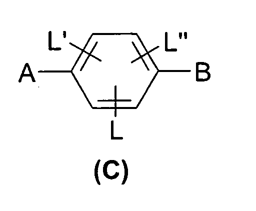 wherein
A and B which may be the same or different are a hydroxy group, a nitro group, a carboxyl group, an amino group, a straight chain or branched C₁₋₆ alkyl group, a straight chain or branched C₁₋₆ alkoxy group, a straight chain or branched C₁₋₇ alkylcarbonyl group, straight chain or branched C₁₋₇ alkoxycarbonyl group or a straight chain or branched C₁₋₆ alkylamino group, or represented by the following formula (1-1) 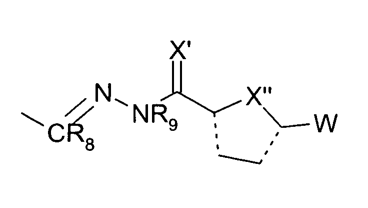
wherein
the dotted lines independently represent a single bond or a double bond,
R₈ and R₉ independently represent a hydrogen atom or a C₁₋₆ alkyl group,
X'and X" are independently O or S,
W is a hydrogen atom, nitro group, a cyano group, a carboxyl group or a group of the formula -COZ'R₁₀,
wherein
Z' is O or S or NH, and
R₁₀ is a C₁₋₆ alkyl group; and
L, L' and L" which may be the same or different represent
a hydrogen atom,
a hydroxy group,
an alkyl group,
an alkoxy group,
a halogen atom,
a carboxyl group,
an alkylcarbonyl group,
an alkoxycarbonyl group,
an amino group,
an alkylamino group, or
a dialkylamino group,
provided that at least one of A and B is represented by formula (1-1).

10. The compound of claim 9, wherein A is hydrogen or a hydroxyl group.

11. The compound of claim 9 or 10 whereinX' and X" are oxygen atoms.

12. The compound of claim 9, 10 or 11 wherein L is a hydroxyl group.

13. The compound according to any one of claims 9 to 12, wherein W is a nitro group.

14. The 5H-Thiazolo[3,2]pyrimidine derivative or a salt or ester thereof according to claim 1 for use as a medicine, which is represented by the following formula (I): 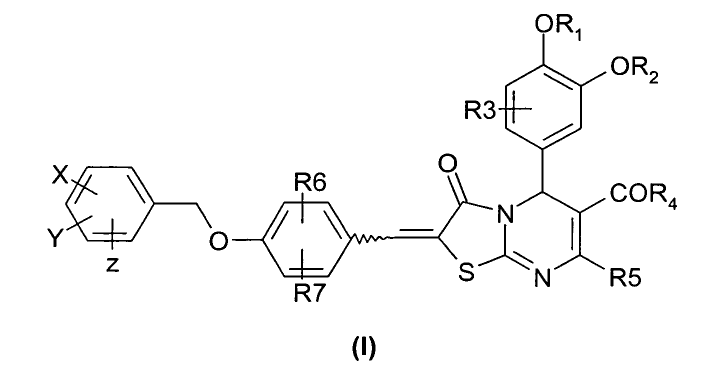 wherein
R₁ and R₂ which may be the same or different, represent a straight chain or branched alkyl group,
a straight chain or branched alkenyl group,
a cycloalkyl group,
an aryl group, or
R₁ and R₂ together may form an alkylene group;
R₃ represents
a hydrogen atom,
a halogen atom,
a straight chain or branched alkyl group,
R₄ represents
a straight chain or branched alkoxy group,
a straight chain or branched alkyl group,
a cycloalkyloxy group,
an alkylamino group,
a cycloalkylamino group,
R₅ represents
a hydrogen atom,
a straight chain or branched alkyl group,
R₆ and R₇ which may be the same or different represent
a hydrogen atom,
a halogen atom,
a straight chain or branched alkyl group,
a straight chain or branched alkoxy group,
a straight chain or branched alkenyl group,
a cycloalkyl group,
an aryl group,
X, Y, and Z which may be same or different represent
a hydrogen atom,
a halogen atom,
an alkyl group, or
an acyl group.

15. The 5H-Thiazolo[3,2]pyrimidine derivative or a salt or ester thereof for use as a medicine according to claim 14, wherein R₁ and R₂ together form an alkylene group.

16. The 5H-Thiazolo[3,2]pyrimidine derivative or a salt or ester thereof for use as a medicine according to claim 14 or 15, wherein R₃ represents a hydrogen atom.

17. The 5H-Thiazolo[3,2]pyrimidine derivative or a salt or ester thereof for use as a medicine according to any one of claims 14 to 16, wherein R₄ represents a straight chain or branched alkoxy group.

18. The 5H-Thiazolo[3,2]pyrimidine derivative or a salt or ester thereof for use as a medicine according to any one of claims 14 to 17, wherein R₅ represents a straight chain or branched alkyl group.

19. The 5H-Thiazolo[3,2]pyrimidine derivative or a salt or ester thereof for use as a medicine according to any one of claims 14 to 18, wherein R₆ and R₇ which may be the same or different represent a halogen atom or a straight chain or branched alkyl group.

20. The 5H-Thiazolo[3,2]pyrimidine derivative or a salt or ester thereof for use as a medicine according to any one of claims 14 to 19, wherein X is a halogen atom and Y and Z represent a hydrogen atom.

21. The 5H-Thiazolo[3,2]pyrimidine derivative or a salt or ester thereof for use as a medicine according to any one of claims 14 to 20, wherein X is a halogen.

22. The 5H-Thiazolo[3,2]pyrimidine derivative or a salt or ester thereof according to claim 2 for use as a medicine, which is represented by the following formula (II): 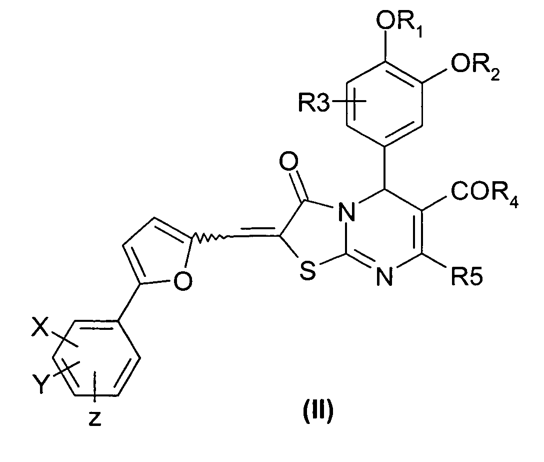 wherein
R₁ and R₂ which may be the same or different, represent
a straight chain or branched alkyl group,
a straight chain or branched alkenyl group,
a cycloalkyl group,
an aryl group, or
R₁ and R₂ together may form an alkylene group;
R₃ represents
a hydrogen atom,
a halogen atom,
a straight chain or branched alkyl group,
R₄ represents
a straight chain or branched alkoxy group,
a straight chain or branched alkyl group,
a cycloalkyloxy group,
an alkylamino group,
a cycloalkylamino group,
R₅ represents
a hydrogen atom,
a straight chain or branched alkyl group,
X, Y, and Z which may be same or different represent
a hydrogen atom,
a carboxyl group
a halogen atom,
a nitro group,
a cyano group, or
an acyl group.

23. The 5H-Thiazolo[3,2]pyrimidine derivative or a salt or ester thereof for use as a medicine according to claim 22, wherein R₁ and R₂ are the same or different and represent an alkyl group or together form an alkylene group.

24. The 5H-Thiazolo[3,2]pyrimidine derivative or a salt or ester thereof for use as a medicine according to claim 22 or 23, wherein R₃ represents a halogen atom.

25. The 5H-Thiazolo[3,2]pyrimidine derivative or a salt or ester thereof for use as a medicine according to any one of claims 22 to 25, wherein R₄ represents a straight chain or branched alkoxy group.

26. The 5H-Thiazolo[3,2]pyrimidine derivative or a salt or ester thereof for use as a medicine according to any one of claims 22 to 25, wherein R₅ represents a straight chain or branched alkyl group.

27. The 5H-Thiazolo[3,2]pyrimidine derivative or a salt or ester thereof for use as a medicine according to any one of claims 22 to 26, wherein X is a carboxyl group and Y and Z represent a hydrogen atom.

28. Compound according to claim 3 for use as a medicine, or a salt or ester thereof, wherein the compound is represented by the following formula (III): 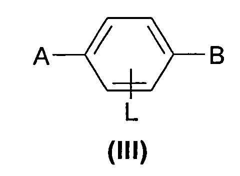 wherein
A and B which may be the same or different are represented by the following formula 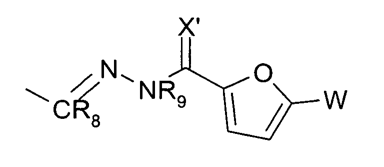
wherein
R₈ and R₉ independently represent a hydrogen atom or a C₁₋₆ alkyl group,
X' is O or S,
W is a nitro group, a cyano group, a carboxyl group or a group of the formula-COZ'R₁₀,
wherein
Z' is O or S or NH, and
R₁₀ is a C₁₋₆ alkyl group; and
L represents
a hydrogen atom,
an alkyl group,
an alkoxy group, or
a halogen atom.

29. The compound of claim 28, wherein A and B are the same.

30. The compound according to any one of claims 28 or 29, wherein R₈ is a hydrogen atom.

31. The compound according to any one of claims 28 to 30, wherein R₉ is a hydrogen atom.

32. The compound according to any one of claims 28 to 31, wherein X' is O.

33. The compound according to any one of claims 28 to 32, wherein W is a nitro group.

34. A heterocyclic compound having a molecular weight of less than 1500 dalton according to any one of the preceding claims or a salt thereof for use as a medicine, whereby the compound gives a positive result as an inhibitor in a cell-free screening method for the identification of inhibitors of IkB phosphorylation by IKK-β, whereby the method comprises the following steps:
(a) providing a composition containing a functional IKK-complex;
(b) subjecting a substrate peptide comprising IKK-β phosphorylation domain of IkB of in the presence of the compound to phosphorylation by the functional IKK-complex of step (a) under predetermined conditions;
(c) reacting the phosphorylated substrate peptide of step (b) under predetermined conditions with an antibody specific for the IKK-β phosphorylated domain of the stubstrate peptide,
(d) identifying the compound as an inhibitor when the amount of specifically bonded antibody is lower due to the presence of the compound as compared to the absence of the compound,
in particular a compound selected from the group of Compunds 002, 02, 30, 31, 54, 56, 68, 69, 73, 70, and 82.

35. A pharmaceutical composition comprising the compound according to any one of claims 1 to 33 as an active ingredient.

36. The pharmaceutical according to claim 35 for reducing the release of NF-κB.

37. The pharmaceutical according to any one of claims 35 or 36, which is effective for preventing or treating inflammatory diseases.

38. The pharmaceutical composition according to any one of claims 35 to 37, which is effective for preventing or treating atherosclerosis.

39. Use of a compound according to any one of claims 1 to 33 for the manufacture of a medicine.

40. The use according to claim 39, wherein the medicine is for the treament or prevention of atherosclerosis.
